# EUROPEAN PATENT APPLICATION

(11) **EP 4 725 491 A1**
(43) Date of publication of application: **15.04.2026**
(21) Application number: 24818780.9
(22) Date of filing: 07.06.2024
(51) Int. Cl.: A61K 31/519, C07D 471/04, A61P 35/00

(54) **FGFR4 INHIBITOR COMPOSITION, PREPARATION METHOD THEREFOR AND PHARMACEUTICAL USE THEREOF**

(30) Priority: 09.06.2023 CN 202310684931
(71) Applicant: Abbisko Therapeutics Co., Ltd., Shanghai 201203 (CN)
(72) Inventor: GAO, Min, Shanghai 201203 (CN); ZHANG, Zhen, Shanghai 201203 (CN); YU, Hongping, Shanghai 201203 (CN)
(74) Representative: dompatent
(86) International application number: PCT/CN2024/098046
(87) International publication number: WO 2024/251252

(57) **Abstract**

An FGFR4 inhibitor composition, a preparation method therefor and the pharmaceutical use thereof. The FGFR4 inhibitor composition comprises a free base of the compound as represented by formula (I) having the following structure or an acid salt thereof as an active ingredient; and a preparation process suitable for industrial production is developed by means of using a suitable filler and other pharmaceutically acceptable carriers. A drug preparation with the drug fluidity, the dissolution rate, the content uniformity, the content of related substances, the physical and chemical stability, etc. thereof meeting clinical requirements can be obtained, such that the requirements of clinical research and drug marketing are met, the problem of drug accessibility is solved, and the drug preparation is expected to be quickly developed into a new generation of FGFR4 inhibitors.

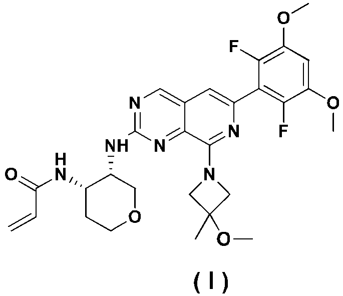

## Description

### TECHNICAL FIELD

The present invention belongs to the field of drug development, and particularly relates to an FGFR4 inhibitor composition, preparation method therefor and pharmaceutical use thereof.

### BACKGROUND

Fibroblast growth factors (FGFs) are a family of 22 structurally related polypeptides with distinct biological activities, and the corresponding receptors of FGFs (FGFRs) belong to a receptor protein tyrosine kinase (RPTK) family. To date, four receptors, i.e., FGFR1, FGFR2, FGFR3 and FGFR4, have been identified, and their interaction with the corresponding ligands (FGFs) leads to receptor dimerization and autophosphorylation, thereby initiating multiple downstream signaling cascades including MAPK and AKT.

Hepatocellular carcinoma (HCC) is one of the leading causes of cancer-related deaths in China and is one of the cancers having the fastest-growing incidence every year. Currently, the first-line treatment regimen employs sorafenib, and there are no approved second-line drugs available. Thus, there is still a need for targeted drugs with anti-tumor agents. 5%-10% of hepatocellular carcinoma patients exhibit overexpression of FGF19, while FGFR4 is the predominant FGFR present in human hepatocytes, and its high expression in hepatocytes is considered to be associated with the aggressiveness of hepatocellular carcinoma. Therefore, FGFR4 plays a very important role in liver cancer. In addition, the interaction of FGF19 and FGFR4 is also thought to be associated with the aggressiveness of other cancer types (e.g., gastric cancer, prostate cancer, lung cancer, colorectal cancer, pancreatic cancer and ovarian cancer).

At present, highly selective FGFR4 inhibitors can effectively treat cancer diseases caused by abnormalities in the FGFR4 signal pathway, and can avoid related side effects such as hyperphosphatemia resulting from FGFR1-3 inhibition. Highly selective small-molecule inhibitors of FGFR4 hold great application prospects in the field of tumor target and immune therapy. FGFR4 inhibitors, as good drug candidates, will meet the demand for targeted drugs for liver cancer and other tumors both domestically and internationally, offering advantages of good safety and stronger specificity.

During the process of long-term research, Abbisko Therapeutics Co., Ltd found a small-molecule compound with a highly selective FGFR4 inhibitory effect (WO2018113584A1), a representative compound of which is shown below:

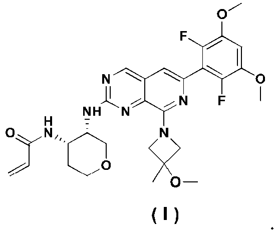

The name of this compound is *N*-((3*S*,4*S*)-3-((6-(2,6-difluoro-3,5-dimethoxyphenyl)-8-(3-methoxy-3-methylazetidin-1-yl)pyrido[3,4-*d*]pyrimidin-2-yl)amino)tetrahydro-2*H*-pyran-4-yl)acrylamide (referred to as the compound of formula (I), or the free base of the compound of formula (I)). This compound can significantly improve the inhibitory effect on the FGFR4 target and the selectivity toward other FGFR1-3 kinase receptors, and thus can be used for treating liver cancer, prostate cancer, pancreatic cancer, esophageal cancer, gastric cancer, lung cancer, breast cancer, ovarian cancer, colon cancer, skin cancer, glioblastoma, rhabdomyosarcoma, etc.

In order to meet the needs of preclinical and clinical formulation development, Abbisko Therapeutics Co., Ltd. has further developed a *p*-toluenesulfonate of the compound of formula (I). This salt-form compound can greatly improve physicochemical properties such as solubility and bioavailability compared to the compound of formula (I) in free form.

However, during the further development of clinical formulations, it was found that the *p-*toluenesulfonate of the compound of formula (I) exhibits poor dissolution properties and inadequate flowability, making industrial production difficult. Therefore, there is an urgent need to optimize formulation formulas and preparation processes to further develop pharmaceutical formulations that can meet the requirements of industrial production in terms of drug dissolution rate, flowability, related substance content, physicochemical stability, etc., thereby satisfying the needs of drug clinical research and marketing.

### SUMMARY

The objective of the present invention is to provide an FGFR4 inhibitor composition to solve the problem of drug accessibility and satisfy the needs of drug clinical research and marketing. Specifically, the inventors improved the material flowability by screening suitable fillers and auxiliary materials, obtained a formulation formula that can be subjected to direct powder filling or wet granulation, and optimized the filling or wet granulation process, so that the uniformity of the drug was greatly improved, and the dissolution properties of the formulation met the clinical requirements. The powder filling process or the wet granulation process adopted by the present invention is simple, exhibits stable quality and high efficiency, and is suitable for industrial production, thereby solving the problem of drug accessibility and satisfying the needs of drug clinical research and marketing.

In a first aspect, the present invention provides a pharmaceutical composition, which comprises a free base of a compound of formula (I) or an acidic salt thereof as an active ingredient, a pharmaceutically acceptable filler and an additional pharmaceutically acceptable carrier, 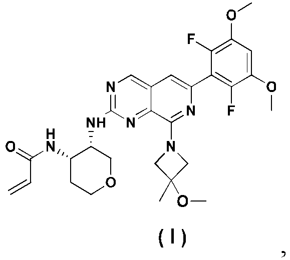 wherein the pharmaceutically acceptable filler is one or more of calcium hydrogen phosphate, mannitol, lactose, microcrystalline cellulose and silicified microcrystalline cellulose.

As a preferred embodiment, the content of the active ingredient is 0.1-60 wt% of the total weight of the pharmaceutical composition, as calculated based on a *p*-toluenesulfonate monohydrate of the compound of formula (I).

As a further preferred embodiment, the content of the active ingredient is 1.0-50 wt% of the total weight of the pharmaceutical composition, as calculated based on the *p*-toluenesulfonate monohydrate of the compound of formula (I).

As a more further preferred embodiment, the content of the active ingredient is 5.0-40 wt% of the total weight of the pharmaceutical composition, as calculated based on the *p-*toluenesulfonate monohydrate of the compound of formula (I).

As a preferred embodiment, the additional pharmaceutically acceptable carrier is one or more of a glidant, a lubricant, a binder and a disintegrant.

As a preferred embodiment, the glidant is colloidal silica, and the content of the glidant is 0.1-5.0 wt% of the total weight of the pharmaceutical composition.

As a further preferred embodiment, the content of the glidant is 0.5-4.0 wt% of the total weight of the pharmaceutical composition.

As a more further preferred embodiment, the content of the glidant is 0.8-2.0 wt% of the total weight of the pharmaceutical composition.

As a preferred embodiment, the lubricant is magnesium stearate and/or sodium stearyl fumarate, and the content of the lubricant is 0.1-5.0 wt% of the total weight of the pharmaceutical composition.

As a further preferred embodiment, the content of the lubricant is 0.2-3.0 wt% of the total weight of the pharmaceutical composition.

As a more further preferred embodiment, the content of the lubricant is 0.3-1.5 wt% of the total weight of the pharmaceutical composition.

As a more further preferred embodiment, the content of the lubricant is 0.3-1.0 wt% of the total weight of the pharmaceutical composition.

As a preferred embodiment, the binder is hydroxypropyl cellulose, methyl cellulose, hydroxypropyl methyl cellulose, polyvinylpyrrolidone, povidone and/or sodium carboxymethyl starch, and the content of the binder is 0.0-20.0 wt% of the total weight of the pharmaceutical composition.

As a further preferred embodiment, the content of the binder is 0.0-10.0 wt% of the total weight of the pharmaceutical composition.

As a more further preferred embodiment, the content of the binder is 0.0-4.0 wt% of the total weight of the pharmaceutical composition.

As a preferred embodiment, the disintegrant is croscarmellose sodium and/or sodium carboxymethyl starch, and the content of the disintegrant is 0.5-20.0 wt% of the total weight of the pharmaceutical composition.

As a further preferred embodiment, the content of the disintegrant is 1.0-10.0 wt% of the total weight of the pharmaceutical composition.

As a more further preferred embodiment, the content of the disintegrant is 2.0-6.0 wt% of the total weight of the pharmaceutical composition.

As a preferred embodiment, the pharmaceutically acceptable filler is anhydrous calcium hydrogen phosphate.

As a preferred embodiment, the pharmaceutically acceptable filler is one or more of mannitol and microcrystalline cellulose.

As a more preferred embodiment, the pharmaceutically acceptable filler is a mixture of mannitol and microcrystalline cellulose.

As a more preferred embodiment, mannitol and microcrystalline cellulose in the pharmaceutically acceptable filler are in a mass ratio of 1:(1±0.2), preferably 1:1.

As a preferred embodiment, the content of the pharmaceutically acceptable filler is 1.0-98.0 wt% of the total weight of the pharmaceutical composition.

As a further preferred embodiment, the content of the pharmaceutically acceptable filler is 20.0-95.0 wt% of the total weight of the pharmaceutical composition.

As a more further preferred embodiment, the content of the pharmaceutically acceptable filler is 40.0-90.0 wt% of the total weight of the pharmaceutical composition.

As a more further preferred embodiment, the content of the pharmaceutically acceptable filler is 50.0-70 wt% of the total weight of the pharmaceutical composition.

As a further preferred embodiment, a unit dosage form of the pharmaceutical composition comprises the active ingredient, a glidant, a lubricant, a binder and a disintegrant,
wherein the content of the active ingredient is 5.0-40.0 wt% of the total weight of the pharmaceutical composition, and/or
the content of the glidant is 0.8-2.0 wt% of the total weight of the pharmaceutical composition, and/or
the content of the lubricant is 0.3-1.0 wt% of the total weight of the pharmaceutical composition, and/or
the content of the binder is 0.0-4.0 wt% of the total weight of the pharmaceutical composition, and/or
the content of the disintegrant is 2.0-6.0 wt% of the total weight of the pharmaceutical composition.

As a more further preferred embodiment, a unit dosage form of the pharmaceutical composition comprises the active ingredient, colloidal silica, magnesium stearate, hydroxypropyl cellulose and croscarmellose sodium,
wherein the content of the active ingredient is 5.0-40.0 wt% of the total weight of the pharmaceutical composition, and/or
the content of colloidal silica is 0.8-2.0 wt% of the total weight of the pharmaceutical composition, and/or
the content of magnesium stearate is 0.3-1.0 wt% of the total weight of the pharmaceutical composition, and/or
the content of hydroxypropyl cellulose is 0.0-4.0 wt% of the total weight of the pharmaceutical composition, and/or
the content of croscarmellose sodium is 2.0-6.0 wt% of the total weight of the pharmaceutical composition.

As a preferred embodiment, the unit dosage form of the pharmaceutical composition further comprises a filler, wherein the content of the filler is 40.0-90.0 wt% of the total weight of the pharmaceutical composition.

As a preferred embodiment, the unit dosage form of the pharmaceutical composition further comprises calcium hydrogen phosphate as a filler, wherein the content of the calcium hydrogen phosphate is 40.0-90.0 wt% of the total weight of the pharmaceutical composition.

As a preferred embodiment, the unit dosage form of the pharmaceutical composition further comprises mannitol and microcrystalline cellulose as fillers, wherein the content of mannitol and microcrystalline cellulose is 40.0-90.0 wt% of the total weight of the pharmaceutical composition, wherein mannitol and microcrystalline cellulose are in a mass ratio of 1:1.

As a preferred embodiment, when the sum of the contents of the components in the pharmaceutical composition is less than 100%, the content of the filler is adjusted such that the sum of the contents of the components in the pharmaceutical composition is 100 wt%.

As a preferred embodiment, the sum of the contents of the components in the pharmaceutical composition is 100 wt%.

As a preferred embodiment, the active ingredient is an anhydrate, a hydrate or a solvate of the free base of the compound of formula (I) or the acidic salt thereof.

As a further preferred embodiment, the active ingredient is a solvate of the free base of the compound of formula (I) or the acidic salt thereof, wherein the solvent is selected from the group consisting of organic solvents including alcohols, chloroalkanes, ketones, ethers, cyclic ethers, esters, alkanes, cycloalkanes, benzenes, amides and sulfoxides, and a mixture thereof and an aqueous solution thereof.

As a more further preferred embodiment, the active ingredient is a solvate of the free base of the compound of formula (I) or the acidic salt thereof, wherein the solvent is selected from the group consisting of methanol, ethanol, *n*-propanol, isopropanol, dichloromethane, acetonitrile, acetone, 1,4-dioxane, tetrahydrofuran, *N,N*-dimethylformamide, ethyl acetate, isopropyl acetate, methyl *tert-*butyl ether and 2-methoxyethyl ether, and a mixture thereof and an aqueous solution thereof.

As a further preferred embodiment, the active ingredient is an anhydrate or a hydrate of the free base of the compound of formula (I) or the acidic salt thereof.

As a more further preferred embodiment, the active ingredient is an anhydrate of the free base of the compound of formula (I) or the acidic salt thereof.

As a more further preferred embodiment, the active ingredient is a hydrate of the free base of the compound of formula (I) or the acidic salt thereof, wherein the hydrate comprises 1-3 water molecules per molecule.

As the most preferred embodiment, the active ingredient is a hydrate of the free base of the compound of formula (I) or the acidic salt thereof, wherein the hydrate comprises 1 water molecule per molecule.

As a more further preferred embodiment, the active ingredient is a hydrate of *p-*toluenesulfonate of the compound of formula (I), wherein the hydrate comprises 1-3 water molecules per molecule.

As the most preferred embodiment, the active ingredient is a hydrate of *p*-toluenesulfonate of the compound of formula (I), wherein the hydrate comprises 1 water molecule per molecule.

As a preferred embodiment, the active ingredient is a *p*-toluenesulfonate monohydrate of the compound of formula (I).

As a further preferred embodiment, an X-ray powder diffraction pattern of the *p-*toluenesulfonate monohydrate of the compound of formula (I) comprises 5 or more peaks at diffraction angles (2θ) selected from the group consisting of 9.02±0.2°, 10.60±0.2°, 12.04±0.2°, 12.72±0.2°, 15.32±0.2°, 17.18±0.2°, 17.68±0.2°, 18.56±0.2°, 19.60±0.2°, 20.60±0.2°, 21.22±0.2°, 22.90±0.2°, 23.82±0.2°, 24.12±0.2°, 24.60±0.2°, 24.90±0.2°, 25.58±0.2°, 27.78±0.2°, 29.04±0.2°, 31.48±0.2°, 36.60±0.2° and 38.16±0.2°.

As a further preferred embodiment, the X-ray powder diffraction pattern of the *p-*toluenesulfonate monohydrate of the compound of formula (I) comprises 5 or more peaks at diffraction angles (2θ) selected from the group consisting of 17.68±0.2°, 22.9±0.2°, 19.6±0.2°, 17.18±0.2°, 9.02±0.2°, 24.9±0.2°, 12.72±0.2°, 21.22±0.2°, 18.56±0.2°, 10.6±0.2°, 23.82±0.2°, 24.12±0.2°, 24.6±0.2°, 25.58±0.2° and 15.32±0.2°.

As a further preferred embodiment, the X-ray powder diffraction pattern of the *p-*toluenesulfonate monohydrate of the compound of formula (I) comprises characteristic peaks at 2θ angles shown in Table A.

**Table A**

| 2θ (°) | 2θ (°) | 2θ (°) |
|---|---|---|
| 9.02 | 23.82 | 32.38 |
| 10.60 | 24.12 | 32.84 |
| 12.04 | 24.60 | 34.04 |
| 12.72 | 24.90 | 35.10 |
| 14.88 | 25.58 | 36.60 |
| 15.32 | 26.20 | 37.10 |
| 17.18 | 26.66 | 38.16 |
| 17.68 | 27.78 | 39.72 |
| 18.56 | 29.04 | 40.14 |
| 19.60 | 29.51 | 41.06 |
| 20.60 | 30.62 | 42.38 |
| 21.22 | 31.48 | 42.72 |
| 22.90 | 31.92 | 44.24 |

As a preferred embodiment, the active ingredient is a *p*-toluenesulfonate monohydrate of the compound of formula (I), having a particle size (D90) of 5-330 µm.

As a further preferred embodiment, the active ingredient is a *p*-toluenesulfonate monohydrate of the compound of formula (I), having a particle size (D90) of 10-200 µm.

As a more further preferred embodiment, the active ingredient is a *p*-toluenesulfonate monohydrate of the compound of formula (I), having a particle size (D90) of 20-150 µm.

As a preferred embodiment, the active ingredient is a *p*-toluenesulfonate monohydrate of the compound of formula (I), having a particle size (D50) of 2-200 µm.

As a further preferred embodiment, the active ingredient is a *p*-toluenesulfonate monohydrate of the compound of formula (I), having a particle size (D50) of 4-100 µm.

As a more further preferred embodiment, the active ingredient is a *p-*toluenesulfonate monohydrate of the compound of formula (I), having a particle size (D50) of 6-50 µm.

As a more further preferred embodiment, the active ingredient is a *p*-toluenesulfonate monohydrate of the compound of formula (I), having a particle size (D50) of 6-25 µm.

As a preferred embodiment, when the sum of the contents of the components in the pharmaceutical composition is less than 100%, the content of the filler is adjusted such that the sum of the contents of the components in the pharmaceutical composition is 100 wt%.

As a further preferred embodiment, the sum of the contents of the active ingredient, the pharmaceutically acceptable filler, the glidant, the lubricant, the binder and the disintegrant is 100 wt%.

As a preferred embodiment, the pharmaceutical composition is obtained by a direct powder filling, dry granulation or wet granulation process.

As a further preferred embodiment, the pharmaceutical composition is obtained by a wet granulation process.

As a preferred embodiment, the pharmaceutical composition is in a capsule or a tablet form.

As a further preferred embodiment, a unit dosage form of the pharmaceutical composition comprises 1-500 mg of the active ingredient, as calculated based on the free base of the compound of formula (I).

As a more further preferred embodiment, the unit dosage form of the pharmaceutical composition comprises 1-200 mg of the active ingredient, as calculated based on the free base of the compound of formula (I).

As a still more further preferred embodiment, the unit dosage form of the pharmaceutical composition comprises 1 mg, 5 mg, 20 mg, 50 mg, 60 mg, 80 mg, 100 mg or 200 mg of the active ingredient, as calculated based on the free base of the compound of formula (I).

In a second aspect, the present invention provides a preparation method for the pharmaceutical composition according to the first aspect of the present invention, which comprises the following step(s): mixing the free base of the compound of formula (I) or the acidic salt thereof as the active ingredient with the pharmaceutically acceptable filler to obtain the pharmaceutical composition, 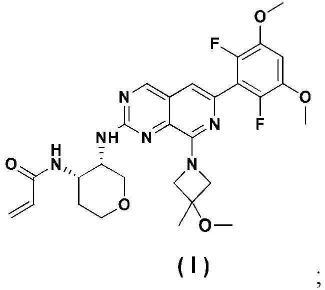 and
**optionally,** adding the additional pharmaceutically acceptable carrier before, during or after the mixing; and
**optionally,** subjecting the pharmaceutical composition prepared above to powder filling, dry granulation or wet granulation to obtain a capsule or tableting to obtain a tablet.

As a preferred embodiment, the preparation method comprises mixing the free base of the compound of formula (I) or the acidic salt thereof with the pharmaceutically acceptable filler to obtain the pharmaceutical composition, and adding the additional pharmaceutically acceptable carrier before or during the mixing.

As a preferred embodiment, the preparation method comprises mixing the free base of the compound of formula (I) or the acidic salt thereof with the pharmaceutically acceptable filler to obtain the pharmaceutical composition, and adding the additional pharmaceutically acceptable carrier after the mixing.

As a further preferred embodiment, the preparation method comprises mixing the free base of the compound of formula (I) or the acidic salt thereof with the pharmaceutically acceptable filler to obtain the pharmaceutical composition, and adding the additional pharmaceutically acceptable carrier before, during or after the mixing.

As a further preferred embodiment, in the preparation method, the active ingredient of the pharmaceutical composition is separately subjected to a milling pretreatment before the mixing.

As a more further preferred embodiment, the preparation method comprises subjecting the pharmaceutical composition prepared above to filling to obtain a capsule.

As a more further preferred embodiment, the preparation method comprises subjecting the pharmaceutical composition prepared above to tableting to obtain a tablet.

As a preferred embodiment, the additional pharmaceutically acceptable carrier is one or more of a glidant, a lubricant, a binder and a disintegrant.

As a further preferred embodiment, the preparation method comprises mixing a *p-*toluenesulfonate monohydrate of the compound of formula (I) with calcium hydrogen phosphate, croscarmellose sodium, colloidal silica and/or an additional pharmaceutically acceptable carrier to obtain the pharmaceutical composition.

As a more further preferred embodiment, the preparation method comprises mixing a *p-*toluenesulfonate monohydrate of the compound of formula (I) with calcium hydrogen phosphate, croscarmellose sodium, colloidal silica and/or an additional pharmaceutically acceptable carrier to obtain a pharmaceutical composition; and
further mixing magnesium stearate and/or sodium stearyl fumarate with the aforementioned pharmaceutical composition to obtain the pharmaceutical composition.

As a further preferred embodiment, the preparation method comprises mixing a *p-*toluenesulfonate monohydrate of the compound of formula (I) with mannitol, microcrystalline cellulose, a first portion of croscarmellose sodium and/or an additional pharmaceutically acceptable carrier to obtain the pharmaceutical composition.

As a more further preferred embodiment, the preparation method comprises: mixing a *p-*toluenesulfonate monohydrate of the compound of formula (I) with mannitol, microcrystalline cellulose, a first portion of croscarmellose sodium and/or an additional pharmaceutically acceptable carrier to obtain the pharmaceutical composition; and adding an aqueous solution of hydroxypropyl cellulose, performing wet granulation, and then drying.

As a still more further preferred embodiment, the preparation method comprises: mixing a*p-*toluenesulfonate monohydrate of the compound of formula (I) with mannitol, microcrystalline cellulose, a first portion of croscarmellose sodium and/or an additional pharmaceutically acceptable carrier to obtain the pharmaceutical composition; adding an aqueous solution of hydroxypropyl cellulose, performing wet granulation, and then drying; and adding colloidal silica and a second portion of croscarmellose sodium to further mix with dried granules obtained in the previous step.

As a still more further preferred embodiment, the preparation method comprises: mixing a*p-*toluenesulfonate monohydrate of the compound of formula (I) with mannitol, microcrystalline cellulose, a first portion of croscarmellose sodium and/or an additional pharmaceutically acceptable carrier to obtain a pharmaceutical composition; adding an aqueous solution of hydroxypropyl cellulose, performing wet granulation, and then drying; extragranularly adding colloidal silica and a second portion of croscarmellose sodium to further mix with dried granules obtained in the previous step; and adding magnesium stearate and performing final mixing to obtain the pharmaceutical composition.

As a more further preferred embodiment, before and/or after the mixing, the components of the pharmaceutical composition are sieved individually or as a mixture through a 30- to 100-mesh sieve.

As a more further preferred embodiment, before and/or after the mixing, the components of the pharmaceutical composition are sieved individually or as a mixture through a 30- to 50-mesh sieve.

As a more further preferred embodiment, before and/or after the mixing, the components of the pharmaceutical composition are sieved individually or as a mixture through a 40-mesh sieve.

As a more further preferred embodiment, the mixing of the components of the pharmaceutical composition is performed at a speed of 10-100 rpm.

As a more further preferred embodiment, the mixing of the components of the pharmaceutical composition is performed at a speed of 10-50 rpm.

As a more further preferred embodiment, the mixing of the components of the pharmaceutical composition is performed at a speed of 20-30 rpm.

In a third aspect, the present invention provides a use of a pharmaceutical composition in the preparation of a medicament as an FGFR4 inhibitor.

As a more further preferred embodiment, the use of the pharmaceutical composition is a use in the preparation of a medicament for treating FGFR4-associated liver cancer, prostate cancer, pancreatic cancer, esophageal cancer, gastric cancer, lung cancer, breast cancer, ovarian cancer, colon cancer, skin cancer, glioblastoma or rhabdomyosarcoma.

In a fourth aspect, the present invention provides a method for treating FGFR4-associated liver cancer, prostate cancer, pancreatic cancer, esophageal cancer, gastric cancer, lung cancer, breast cancer, ovarian cancer, colon cancer, skin cancer, glioblastoma or rhabdomyosarcoma, which comprises administering to a patient in need thereof a therapeutically effective amount of the pharmaceutical composition.

In a fifth aspect, the present invention provides a pharmaceutical composition for use as an FGFR4 inhibitor drug.

The pharmaceutical composition of the present invention can be prepared by a direct powder filling process or a wet granulation process and meets the clinical requirements in terms of drug dissolution rate, content uniformity, related substance content, physicochemical stability, etc., thereby satisfying the needs of clinical research and drug marketing. Especially, the wet granulation process is suitable for industrialization, solving the problem of drug accessibility.

### BRIEF DESCRIPTION OF THE DRAWINGS

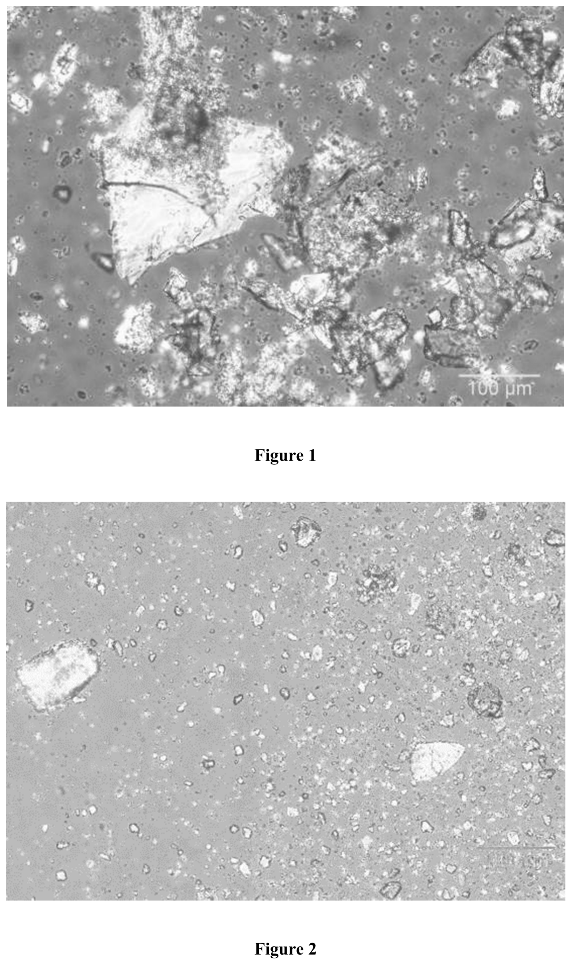

FIG. 1 shows the morphology of the unmilled active pharmaceutical ingredient.
FIG. 2 shows the morphology of the active pharmaceutical ingredient in Test 1.

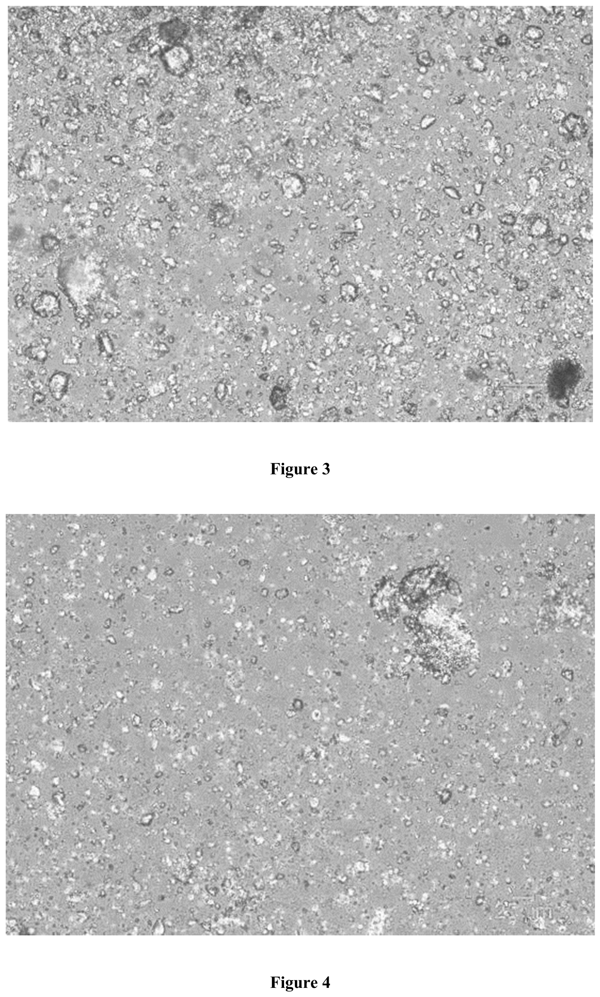

FIG. 3 shows the morphology of the active pharmaceutical ingredient in Test 2.
FIG. 4 shows the morphology of the active pharmaceutical ingredient in Test 3.
FIG. 5 shows the dissolution profiles of magnesium stearate in different proportions in a direct mixing process, where the abscissa represents time (min), and the ordinate represents dissolution rate (%).
FIG. 6 shows the dissolution profiles of formula and process screening batches, where the abscissa represents time (min), and the ordinate represents dissolution rate (%).

### DETAILED DESCRIPTION OF THE INVENTION

### 1. Specific definitions

Detailed description: Unless otherwise stated or specified, the following terms used in the specification and claims have the following meanings.

As used herein, the term "acidic salt" refers to a pharmaceutically acceptable salt, which is generally an inorganic acid salt or an organic acid salt, as known to those of ordinary skill in the art. The inorganic acid salt may be hydrochloride, sulfate, hydrobromide, hydrofluoride, hydroiodide or phosphate; the organic acid salt may be acetate, dichloroacetate, trichloroacetate, trifluoroacetate, benzenesulfonate, *p*-toluenesulfonate, 4-chlorobenzenesulfonate, 1,5-naphthalenedisulfonate, naphthalene-2-sulfonate, ethane-1,2-disulfonate, methanesulfonate, ethanesulfonate, trifluoromethanesulfonate, benzoate, decanoate, hexanoate, octanoate, cinnamate, citrate, cyclamate, camphorsulfonate, aspartate, camphorate, gluconate, glucuronate, glutamate, erythorbate, lactate, malate, mandelate, pyroglutamate, tartrate, dodecyl sulfate, dibenzoyltartrate, formate, fumarate, galactonate, gentisate, acetohydroxamate, malonate, succinate, glutarate, adipate, sebacate, 2-ketoglutarate, glycolate, hippurate, isethionate, lactobionate, ascorbate, aspartate, laurate, maleate, nicotinate, oleate, orotate, oxalate, succinate, palmitate, pamoate, propionate, 4-acetamidobenzoate, 4-aminobenzoate, salicylate, 4-aminosalicylate, 2,5-dihydroxybenzoate, 1-hydroxy-2-naphthoate, stearate, thiocyanate, undecylenate or succinate.

As used herein, the term "solvate" refers to a compound composed of a solvent and a dissolved substance. The solvent may be an organic solvent or other liquid, while the dissolved substance may be ions, molecules or other substances. The solvent compound may be a solid, liquid or gas. In the context of the present invention, the solvate refers to a complex formed by dissolving an active substance in a solvent, where molecules of the solvent bind to molecules, ions, etc. of the active substance, thereby altering the original state of the active ingredient. The organic solvent includes, but is not limited to: alcohols, chloroalkanes, ketones, ethers, cyclic ethers, esters, alkanes, cycloalkanes, benzenes, amides or sulfoxides, or mixtures thereof or aqueous solutions thereof. Specifically, the organic solvent may be methanol, ethanol, *n-*propanol, isopropanol, dichloromethane, acetonitrile, acetone, 1,4-dioxane, tetrahydrofuran, *N,N*-dimethylformamide, ethyl acetate, isopropyl acetate, methyl *tert-*butyl ether or 2-methoxyethyl ether, a mixture thereof or an aqueous solution thereof.

As used herein, the term "pharmaceutical composition" refers to a mixture containing one or more of the compounds or physiologically/pharmaceutically acceptable crystal forms, salt forms or pro-drugs thereof described herein and other chemical components, as well as other components such as physiologically/pharmaceutically acceptable carriers. The purpose of the pharmaceutical composition is to promote the administration to an organism, facilitate the absorption of the active ingredient, and thereby exert biological activities.

The term "free base of the compound of formula (I)" refers to the compound of formula (I) shown below:

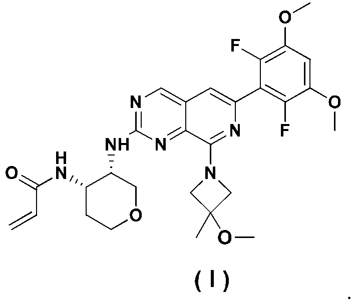

The term "active ingredient" refers to a free base of the compound of formula (I) or an acidic salt thereof; the active ingredient is preferably an anhydrate, a hydrate or a solvate of the free base of the compound of formula (I) or the acidic salt thereof; the solvent is selected from the group consisting of organic solvents including alcohols, chloroalkanes, ketones, ethers, cyclic ethers, esters, alkanes, cycloalkanes, benzenes, amides and sulfoxides, and mixtures thereof and aqueous solutions thereof. Preferably, the organic solvent is selected from the group consisting of methanol, ethanol, *n*-propanol, isopropanol, dichloromethane, acetonitrile, acetone, 1,4-dioxane, tetrahydrofuran, *N,N*-dimethylformamide, ethyl acetate, isopropyl acetate, methyl *tert-*butyl ether and 2-methoxyethyl ether, and mixtures thereof and aqueous solutions thereof.

The term "active pharmaceutical ingredient" or "API" refers to a *p*-toluenesulfonate monohydrate of the compound of formula (I).

The pharmaceutical composition of the present invention may comprise one or more pharmaceutically acceptable carriers, including but not limited to fillers, glidants, binders, foaming agents, lubricants, disintegrants, diluents, stabilizers, buffers, adjuvants, vehicles, emulsifiers, viscosity modifiers, surfactants, preservatives, flavoring agents or coloring agents. For pharmaceutically acceptable excipients, reference may be made to example descriptions in Handbook of Pharmaceutical Excipients by R. C. Rowe and P. J. Sheskey.

As used herein, the term "filler" refers to any pharmaceutically acceptable substance or composition added to a formulation to increase bulk. Suitable fillers include, but are not limited to, mannitol, lactose, microcrystalline cellulose, mannitol, silicified microcrystalline cellulose and calcium hydrogen phosphate.

As used herein, the term "glidant" refers to a compound or composition added to a formulation to improve interactions among various factors, which makes the flowability of the powder better. Suitable glidants include (but are not limited to) colloidal silica and talc.

As used herein, the term "binder" refers to a pharmaceutically acceptable compound or composition added to a formulation to maintain the active pharmaceutical ingredient and inactive ingredients together in the form of a cohesive mixture. Dry binders used for direct compaction must exhibit cohesive and adhesive forces, such that particles coalesce when compacted. Binders used for wet granulation are hydrophilic and water-soluble, and typically dissolve in water to form a wet mass, which is then granulated. Examples of suitable binders include, but are not limited to, povidone, Plasdone K29/32, Plasdone S-630, hydroxypropyl cellulose, methyl cellulose, polyvinylpyrrolidone, aluminum stearate, hydroxypropyl methyl cellulose and analogs thereof. These binders may additionally function as water sequestering agents (e.g., povidone).

As used herein, the term "lubricant" refers to any pharmaceutically acceptable agent that reduces surface friction, lubricates particle surfaces, decreases tendency for static electricity to build up and/or reduces particle friability. Thus, the lubricant may function as an anti-coalescence agent. Conventional lubricants include stearic acid and related compounds, such as magnesium stearate, sodium stearyl fumarate and sodium stearoyl fumarate. Alternative lubricants include glyceryl dibehenate, colloidal silica, talc, other hydrogenated vegetable oils or triglycerides. Examples of suitable alternative lubricants include (but are not limited to) glyceryl dibehenate.

As used herein, the term "disintegrant" refers to a substance added to a composition to help it split (disintegrate) and release a pharmaceutical agent. Examples of disintegrants include (but are not limited to) non-sugar water-soluble polymers, such as: croscarmellose sodium, sodium carboxymethyl starch, crospovidone, sodium starch glycolate and analogs thereof.

As used herein, the terms "D10", "D50" and "D90" are parameters indicating the particle size of the active ingredient, with specific meanings as follows:
D10 refers to the particle size where the cumulative distribution of active ingredient particles is 10%, that is, particles smaller than this particle size account for 10% of all particles by volume content.

D50 refers to the particle size where the cumulative distribution of active ingredient particles is 50%, also called the median diameter or median particle size; it is a typical value indicating the particle size, which accurately divides the entire population into two equal parts; that is, 50% of particles have a particle size larger than this value and the remaining 50% have a particle size smaller than this value. If the D50 of a sample is 5 µm, it means that among all particles constituting the sample, particles having a particle size larger than 5 µm account for 50% and particles having a particle size smaller than 5 µm also account for 50%.

D90 refers to the particle size where the cumulative distribution of active ingredient particles is 90%; that is, particles smaller than this particle size account for 90% of all particles by volume content. This applies similarly to other particle size definitions.

### 2. Analysis method

The determination method for dissolution of capsules in the formulation study of the present invention is shown in the table below:

| **Dissolution apparatus** | Chinese Pharmacopoeia, 2020 Editi**on, 0931, Method 1 (basket method)** |
|---|---|
| Dissolution medium | 0.1 mol/L hydrochloric acid solution |
| Medium volume | 900 mL |
| Water temperature | 37 ± 0.5 °C |
| Rotation speed | 100 rpm (60-75 min is the ultimate rotation speed test, and the rotation speed is 250 rpm) |
| Sampling time point | 45 min (dissolution rate) |
| | 5 min, 15 min, 30 min, 45 min, 60 min and 75 min (dissolution profile) |
| Sampling volume | 5 mL |
| Filtration | PES filter, 0.45 µm |

### 3. Material information

| **No.** | **Component** | **Model** | **Manufacturer** | **Function** |
|---|---|---|---|---|
| 1 | Lactose monohydrate | Flowlac^{®}100 | MEGGLE, Germany | Filler |
| 2 | Mannitol | 100SD | Roquette, French | |
| 3 | Mannitol | 200SD | Roquette, French | |
| 4 | Microcrystalline cellulose | 101 | FMC, USA | |
| 5 | Microcrystalline cellulose | 102 | FMC, USA | |
| 6 | Silicified microcrystalline cellulose | HD90 | JRS | |
| 7 | Anhydrous calcium hydrogen phosphate | Fujicalin(SG) | Fuji Chemical, Japan | |
| 8 | Povidone | K30 | BASF, Germany | Binder |
| 9 | Hydroxypropyl cellulose | EXF | Ashland, USA | |
| 10 | Croscarmellose sodium | - | FMC, USA | Disintegrant |
| 11 | Crospovidone | XL-10 | Ashland, USA | |
| 12 | Sodium carboxymethyl starch | - | Yung Zip Chemical, Japan | |
| 13 | Colloidal silica | 200 | Evonik, Germany | Glidant |
| 14 | Magnesium stearate | - | Mallinckrodt, USA | Lubricant |
| 15 | Sodium stearyl fumarate | - | JRS, Germany | |
| 16 | Empty gelatin capsule | 3# opaque rich yellow | Suzhou Capsugel | Capsule shell |
| 17 | Sodium dodecyl sulfate | - | BASF, Germany | Surfactant |
| 18 | Pharmaceutical high-density polyethylene bottle for oral solid | 45mL/75mL | Sanner Pharmaceutical | Outer packaging |
| 19 | Active pharmaceutical ingredient/API | The free base of the compound of formula (I) was prepared with reference to WO2018113584A1, and the API was prepared according to the method described below. | | Active pharmaceutical ingredient |

The preparation process of the active pharmaceutical ingredient was as follows: 5 mg of the compound of formula (I) in free form was weighed and dissolved in 0.5 mL of ethyl acetate; 1.67 mg of *p*-toluenesulfonic acid was weighed and dissolved in 0.1 mL of tetrahydrofuran; the *p-*toluenesulfonic acid solution was slowly added to the solution of the compound of formula (I) with stirring, the mixture was stirred overnight and filtered, and the filter cake was dried in a vacuum drying oven at 40 °C. The XRPD diffraction angles (2θ) and related intensity data (±0.2°) are as follows:

**Table A**

| 2θ (°) | Intensity % | 2θ (°) | Intensity % | 2θ (°) | Intensity % |
|---|---|---|---|---|---|
| 9.02 | 40.9 | 23.82 | 18.8 | 32.38 | 1.2 |
| 10.60 | 19.6 | 24.12 | 17.7 | 32.84 | 3.7 |
| 12.04 | 7.5 | 24.60 | 14.5 | 34.04 | 2.4 |
| 12.72 | 29.8 | 24.90 | 31.5 | 35.10 | 2.2 |
| 14.88 | 4.3 | 25.58 | 13.8 | 36.60 | 6.9 |
| 15.32 | 12.4 | 26.20 | 4.2 | 37.10 | 3 |
| 17.18 | 47 | 26.66 | 2.9 | 38.16 | 5.1 |
| 17.68 | 100 | 27.78 | 6.8 | 39.72 | 3.3 |
| 18.56 | 23.2 | 29.04 | 8.4 | 40.14 | 2.4 |
| 19.60 | 61.3 | 29.51 | 1.7 | 41.06 | 2.1 |
| 20.60 | 8.1 | 30.62 | 2.8 | 42.38 | 3.5 |
| 21.22 | 29.1 | 31.48 | 8.1 | 42.72 | 2 |
| 22.90 | 84.9 | 31.92 | 2.4 | 44.24 | 1.9 |

The unit cell is P1, a = 9.079 (3) Å, b = 10.561 (3) Å, c = 10.882 (3) Å, and the unit cell volume is 908.1 (4) Å³.

### 4. Equipment information

| **Name** | **Manufacturer** | **Model** |
|---|---|---|
| Small high-speed mill | Beijing Xingshi Lihe | ZN-04AL |
| Polarizing microscope | Nikon | LV 100N POL |
| Particle size analyzer | Malvern | Mastersizer 2000 |
| Dry granulator | Freund-Vector | TF-LAB |
| Wet granulator | GLATT, Germany | TMG 1/6 |

### 5. Determination method for content uniformity

During the filling process, 10-11 capsule samples prepared in Examples 4-6 of the present application were randomly taken; the relative content of each capsule with the labeled amount as 100% was measured separately; the mean value and standard deviation (SD%), as well as the absolute value (A) of the difference between the labeled amount and the mean value, were calculated; the content uniformity CP of the formula of the present application was calculated according to CP = A + 2.2S (CP ≤ 15.0 meets the requirements of the *Chinese Pharmacopoeia*)*.*

**Chromatographic conditions:** Octadecylsilane-bonded silica gel was used as the filler (Waters XBridge^{®} C18, 4.6 × 150 mm, 3.5 µm chromatographic column or chromatographic column with equivalent performance); a 0.1 vol% trifluoroacetic acid solution was used as mobile phase A, and a solution of 0.1 vol% trifluoroacetic acid in acetonitrile was used as mobile phase B; isocratic elution was performed with mobile phase A-mobile phase B (70:30); the detection wavelength was 232 nm, the flow rate was 1.0 mL/min, the column temperature was 40 °C, the sample injection volume was 10 µL, and the run time was 10 min.

### 6. Determination method for related substances

**Chromatographic conditions:** Octadecylsilane-bonded silica gel was used as the filler (Waters SunFire C18, 4.6 × 150 mm, 3.5 µm chromatographic column or chromatographic column with equivalent performance); a 10 mmol/L ammonium formate solution was used as mobile phase A, and acetonitrile was used as mobile phase B; the detection wavelength was 232 nm, the flow rate was 1.0 mL/min, the column temperature was 40 °C, and the sample injection volume was 10 µL. Gradient elution was performed according to the table below, with a run time of 50.0 min and a post-run time of 5 min.

| **Time (min)** | 0.0 | 10.0 | 15.0 | 25.0 | 35.0 | 45.0 | 50.0 |
|---|---|---|---|---|---|---|---|
| **Mobile phase A (%)** | 70 | 50 | 50 | 45 | 25 | 5 | 5 |
| **Mobile phase B (%)** | 30 | 50 | 50 | 55 | 75 | 95 | 95 |

### 7. Determination of hygroscopic weight gain

**Gravimetric method:** The initial weight of a sample was measured, recorded as W1, and then the weight of the sample after being placed under high-humidity conditions for a certain period of time was measured, recorded as W2; the percentage of hygroscopic weight gain was calculated based on the weight difference, using the following calculation formula: (W2 - W1)/W1 * 100%.

The present invention is further explained in detail through the following examples, which are intended to illustrate specific embodiments of the present invention only and should not be construed as limiting the scope of the present invention in any way.

### Example 1

### (Study on Properties of API and Auxiliary Materials)

The inventors first conducted a study on the physicochemical properties of the active pharmaceutical ingredient. The specific procedures were as follows: The active pharmaceutical ingredient and different auxiliary materials were mixed at different ratios and then placed under different conditions; the samples were taken out at different time points and then observed for appearance, hygroscopic weight gain, active pharmaceutical ingredient content, related substance content, etc. The specific experimental design is shown in the following tables.

All the auxiliary materials used are pharmaceutically acceptable auxiliary materials for oral solid formulations, have been included in the current edition of the *Chinese Pharmacopoeia,* and exhibit stable properties. In the following examples, unless otherwise specified, the active pharmaceutical ingredient (or API) used was the same as that in Example 1.

**Table 1. Information on API and auxiliary materials, and corresponding weight ratios**

| **No.** | **Component** | **Model** | **Manufacturer** | **API : auxiliary material** | **Function** |
|---|---|---|---|---|---|
| 1 | Lactose monohydrate | Flowlac^{®}100 | MEGGLE, Germany | 1:5 | Filler |
| 2 | Mannitol | 100SD | Roquette, French | 1:5 | |
| 3 | Microcrystalline cellulose | 102 | FMC, USA | 1:5 | |
| 4 | Anhydrous calcium hydrogen phosphate | Fujicalin(SG) | Fuji Chemical, Japan | 1:5 | |
| 5 | Povidone | K30 | BASF, Germany | 1:1 | Binder |
| 6 | Hydroxypropyl cellulose | EXF | Ashland, USA | 1:1 | |
| 7 | Croscarmellose sodium | - | FMC, USA | 1:1 | Disintegrant |
| 8 | Crospovidone | XL-10 | Ashland, USA | 1:1 | |
| 9 | Sodium carboxymethyl starch | - | Yung Zip Chemical, Japan | 1:1 | |
| 10 | Colloidal silica | 200 | Evonik, Germany | 10:1 | Glidant |
| 11 | Magnesium stearate | - | Mallinckrodt, USA | 10:1 | Lubricant |
| 12 | Sodium stearyl fumarate | - | JRS, Germany | 10:1 | |
| 13 | Empty gelatin capsule | 3# opaque rich yellow | Suzhou Capsugel | 1:1 | Capsule shell |
| 14 | Sodium dodecyl sulfate | - | BASF, Germany | 10:1 | Surfactant |
| 15 | Active pharmaceutical ingredient | Prepared by the same method as above | | - | Active pharmaceutical ingredient |

**Table 2. Experimental conditions and test items**

| **Experimental conditions** | **Packaging material** | **0d** | **10d** | **30d** |
|---|---|---|---|---|
| 60 °C/open | Glass bottle, without stopper | X^{[1]} (2 bottles) | - | X^{[1]} (2 bottles) |
| 25 °C/92.5% RH/open | | | - | X^{[1]} (2 bottles) |
| Light exposure/open | | | X^{[1]} (2 bottles) | - |
| 40 °C/75% RH/open | | | - | X^{[1]} (2 bottles) |
| 25 °C/92.5% RH/open | Glass bottle, without stopper | Y^{[2]} (1 bottle) | | |
| 40 °C/75% RH/open | | Y^{[2]} (1 bottle) | | |

| | | | | |
|---|---|---|---|---|
| Note: ^{[1]}: Test items: X = appearance and content; ^{[2]}: Test item: Y = hygroscopic weight gain. | | | | |

### Test results:

**Table 3. Appearance results of mixtures of active pharmaceutical ingredient and auxiliary materials**

| **No.** | **Auxiliary material** | **Condition** | | | | |
|---|---|---|---|---|---|---|
| | | **0 d** | **40 °C/75% RH** | **Light exposure** | **60 °C** | **25 °C/92.5% RH** |
| | | | **30 d** | **10 d** | **30 d** | **30 d** |
| 1 | Lactose monohydrate | Pale yellow powder | Pale yellow powder | Pale yellow powder | Pale yellow powder | Pale yellow powder |
| 2 | Mannitol 100SD | Pale yellow powder | Pale yellow powder | Pale yellow powder | Pale yellow powder | Pale yellow, slightly agglomerated |
| 3 | Microcrystalline cellulose 102 | Pale yellow powder | Pale yellow powder | Pale yellow powder | Pale yellow powder | Pale yellow powder |
| 4 | Anhydrous calcium hydrogen phosphate | Pale yellow powder | Pale yellow powder | Pale yellow powder | Pale yellow powder | Pale yellow powder |
| 5 | Povidone K30 | Pale yellow powder | Yellow, liquefied, stuck to bottle bottom | Pale yellow powder | Pale yellow powder | Yellow, liquefied, stuck to bottle bottom |
| 6 | Hydroxypropyl cellulose EXF | Pale yellow powder | Yellow, stuck to bottle bottom | Pale yellow powder | Pale yellow, slightly agglomerated | Yellow, liquefied, stuck to bottle bottom |
| 7 | Croscarmellose sodium | Pale yellow powder | Pale yellow, slightly agglomerated | Pale yellow powder | Pale yellow powder | Pale yellow, stuck to bottle bottom |
| 8 | Crospovidone XL-10 | Pale yellow powder | Pale yellow, stuck to bottle bottom | Pale yellow powder | Pale yellow powder | Pale yellow, stuck to bottle bottom |
| 9 | Sodium carboxymethyl starch | Pale yellow powder | Pale yellow, stuck to bottle bottom | Pale yellow powder | Pale yellow powder | Pale yellow, stuck to bottle bottom |
| 10 | Colloidal silica | Pale yellow powder | Pale yellow powder | Pale yellow powder | Yellow powder | Pale yellow powder |
| 11 | Magnesium stearate | Pale yellow powder | Pale yellow, slightly agglomerated | Pale yellow powder | Pale yellow powder | Pale yellow powder |
| 12 | Sodium stearyl fumarate | Pale yellow powder | Pale yellow powder | Pale yellow powder | Pale yellow powder | Pale yellow powder |
| 13 | Empty gelatin capsule 3# rich yellow | Yellow powder | Yellow powder | Yellow powder | Yellow powder | Yellow, slightly agglomerated |
| 14 | Sodium dodecyl sulfate | Pale yellow powder | Pale yellow powder | Pale yellow powder | Pale yellow powder | Pale yellow powder |
| 15 | Active pharmaceutical ingredient | Yellow powder | Yellow powder | Yellow powder | Yellow powder | Yellow powder |

**Table 4. Percentage changes in hygroscopic weight gain of samples obtained by mixing the active pharmaceutical ingredient with the following auxiliary materials**

| **No.** | **Sample** | **Condition** | | | |
|---|---|---|---|---|---|
| | | **40 °C/75% RH** | | **25 °C/92.5% RH** | |
| | | **10 d (%)** | **30 d (%)** | **10 d (%)** | **30 d (%)** |
| 1 | Lactose monohydrate | 0.1 | -0.1 | 0.2 | 0.3 |
| 2 | Mannitol 100SD | 0.2 | 0.3 | 0.8 | 0.9 |
| 3 | Microcrystalline cellulose 102 | 2.1 | 1.7 | 6.1 | 6.4 |
| 4 | Anhydrous calcium hydrogen phosphate | 1.0 | 0.6 | 1.9 | 3.9 |
| 5 | Povidone K30 | 6.4 | 6.4 | 24.9 | 23.7 |
| 6 | Hydroxypropyl cellulose EXF | 3.7 | 3.7 | 12.9 | 12.7 |
| 7 | Croscarmellose sodium | 9.3 | 8.6 | 26.6 | 26.3 |
| 8 | Crospovidone XL-10 | 4.7 | 4.7 | 16.2 | 15.8 |
| 9 | Sodium carboxymethyl starch | 11.5 | 11.1 | 31.0 | 32.0 |
| 10 | Colloidal silica | 0.5 | 0.4 | 0.5 | 0.9 |
| 11 | Magnesium stearate | 0.3 | 0.2 | 0.6 | 0.4 |
| 12 | Sodium stearyl fumarate | 0.2 | -0.1 | 0.6 | 0.4 |
| 13 | Empty gelatin capsule 3# rich yellow | 2.5 | 2.3 | 12.3 | 11.5 |
| 14 | Sodium dodecyl sulfate | 0.5 | 0.1 | 2.3 | 2.5 |
| 15 | Active pharmaceutical ingredient | 0.2 | -0.3 | 0.6 | 0.3 |

**Table 5. Content determination results of samples obtained by mixing the active pharmaceutical ingredient with the following auxiliary materials**

| No. | Sample | Condition | | | | |
|---|---|---|---|---|---|---|
| | | 0 d (%) | 40 °C/ 75% RH | Light exposure | 60 °C | 25 °C/ 92.5% RH |
| | | | 30 d (%) | 10 d (%) | 30 d (%) | 30 d (%) |
| 1 | Lactose monohydrate FLOWLAC^{®}100 | 101.5 | 98.4 | 98.2 | 98.9 | 98.4 |
| 2 | Mannitol 100SD | 101.3 | 98.2 | 98.2 | 97.1 | 102.5 |
| 3 | Microcrystalline cellulose 102 | 101.9 | 99.1 | 98.8 | 100.5 | 99.8 |
| 4 | Anhydrous calcium hydrogen phosphate | 101.8 | 98.6 | 98.4 | 98.1 | 99.9 |
| 5 | Povidone K30 | 101.4 | 97.4 | 98.5 | 98.2 | 99.8 |
| 6 | Hydroxypropyl cellulose | 102.0 | 97.9 | 98.1 | 97.8 | 99.6 |
| 7 | Croscarmellose sodium | 103.2 | 98.3 | 98.1 | 97.4 | 100.3 |
| 8 | Crospovidone XL-10 | 103.1 | 98.4 | 98.8 | 100.1 | 99.8 |
| 9 | Sodium carboxymethyl starch | 101.7 | 97.8 | 98.3 | 98.4 | 100.2 |
| 10 | Colloidal silica | 102.2 | 98.4 | 98.6 | 98.8 | 99.8 |
| 11 | Magnesium stearate | 102.6 | 98.1 | 98.4 | 97.6 | 99.5 |
| 12 | Sodium stearyl fumarate | 103.1 | 98.3 | 98.7 | 98.4 | 98.8 |
| 13 | Empty gelatin capsule 3# rich yellow | 101.6 | 98.3 | 98.6 | 99.1 | 99.3 |
| 14 | Sodium dodecyl sulfate | 100.8 | 98.4 | 98.4 | 99.5 | 99.6 |
| 15 | API | 100.5 | 98.6 | 96.5 | 102.5 | 99.5 |

| | | | | | | |
|---|---|---|---|---|---|---|
| **Note: "d" in the above table is an abbreviation for day, and it carries the same meaning in the following tables. The related substance results are shown in the summary results of the compatibility between the active pharmaceutical ingredient and various auxiliary materials in Example 2.** | | | | | | |

Under the above different placement conditions, the active pharmaceutical ingredient and different auxiliary materials were mixed at different ratios. The changes in the related substance content are as follows:
(1) The active pharmaceutical ingredient alone was not highly hygroscopic; however, the active pharmaceutical ingredient showed a slight increase in impurities when exposed to 25 °C/high humidity (92.5% RH) conditions, while under high temperature (60 °C), light exposure, and 40 °C/75% RH open conditions, the impurities in the active pharmaceutical ingredient remained stable. This suggests that sealed storage of the product is conducive to increasing the stability of the active pharmaceutical ingredient.
(2) The binary mixture of the active pharmaceutical ingredient and each auxiliary material showed comparable impurity levels to those of the active pharmaceutical ingredient alone under 25 °C/92.5% RH and 40 °C/75% RH open conditions, indicating that all the auxiliary materials did not affect the chemical stability of the active pharmaceutical ingredient under these conditions, exhibiting good compatibility with minimal impact on the appearance and hygroscopicity.
(3) When the binary mixture of the active pharmaceutical ingredient and povidone K30 or hydroxypropyl cellulose EXF or crospovidone XL-10 was placed at 60 °C, a single impurity (RRT = 0.30) was found to increase significantly. Considering the impact of these two auxiliary materials on the chemical stability of the active pharmaceutical ingredient under high-temperature conditions, therefore, the application of these two auxiliary materials in the formula does not represent a preferred option.
(4) After 10 days of exposure to light, the following main impurity generation was observed:
   a) a single impurity (RRT = 0.44) in the active pharmaceutical ingredient increased from <0.05% to 0.07%;
   b) a single impurity (RRT = 0.44) in the binary mixture of the active pharmaceutical ingredient and anhydrous calcium hydrogen phosphate increased from < 0.05% to 0.17%;
   c) a single impurity (RRT = 0.27) in the binary mixture of the active pharmaceutical ingredient and croscarmellose sodium increased from < 0.05% to 0.14%, and another single impurity (RRT = 0.44) increased from < 0.05% to 0.13%; and
   d) a single impurity (RRT = 0.27) in the binary mixture of the active pharmaceutical ingredient and sodium carboxymethyl starch increased from <0.05% to 0.10%, and another single impurity (RRT = 0.44) increased from <0.05% to 0.13%.

The above three binary mixtures showed comparable impurity levels to those of the active pharmaceutical ingredient alone under other conditions, indicating applicability in formulation development. However, this suggests that the products should be protected from light, and attention should be paid to the stability data of subsequent products.

### Example 2

### (Screening of Pharmaceutical Auxiliary Materials)

In this example, items such as material flowability and capsule dissolution rate were used as main evaluation indicators, capsule dosage form and pharmaceutically acceptable pharmaceutical auxiliary materials exhibiting good compatibility with the active pharmaceutical ingredient were adopted, and formula screening was performed within conventional usage ranges, so as to optimize auxiliary material types.

### I. Filler screening

A combination of microcrystalline cellulose 102 and anhydrous calcium hydrogen phosphate or anhydrous calcium hydrogen phosphate alone was selected as a filler. The effects of different fillers on the flowability and dissolution were primarily investigated.

The compatibility results of a binary mixture of the active pharmaceutical ingredient and each filler at a 1:5 weight ratio were used as the basis for filler type screening.

The specific formula composition, dissolution test results, micromeritic properties of final mixed powders, and results of the compatibility between the active pharmaceutical ingredient and fillers are shown in the following tables, respectively.

**Table 6. Formula composition of active pharmaceutical ingredient capsules with different fillers**

| Component | mg/capsule | wt% | mg/capsule | wt% | Function |
|---|---|---|---|---|---|
| Active pharmaceutical ingredient | 133.29 | 38.08 | 133.29 | 38.08 | Active pharmaceutical ingredient |
| Anhydrous calcium hydrogen phosphate | 141.71 | 40.49 | 200.96 | 57.42 | Filler |
| Microcrystalline cellulose 102 | 59.25 | 16.93 | / | / | Filler |
| Croscarmellose sodium | 10.50 | 3.00 | 10.50 | 3.00 | Disintegrant |
| Colloidal silica 200 | 3.50 | 1.00 | 3.50 | 1.00 | Glidant |
| Magnesium stearate | 1.75 | 0.50 | 1.75 | 0.50 | Lubricant |
| Total content | 350.00 | 100.00 | 350.00 | 100 | - |
| 0# empty gelatin capsule | 96±6 | - | 96±6 | - | Capsule shell |

**Table 7. Dissolution test results of capsules with different fillers**

| **Filler** | **Dissolution rate (%)** | | | | | |
|---|---|---|---|---|---|---|
| | **5 min** | **15 min** | **30 min** | **45 min** | **60 min** | **75 min** |
| Combination of microcrystalline cellulose 102 and anhydrous calcium hydrogen phosphate | 59 | 78 | 86 | 91 | 93 | 104 |
| Anhydrous calcium hydrogen phosphate | 61 | 85 | 95 | 98 | 99 | 103 |

**Table 8. Micromeritic property data of final mixed powders with different fillers**

| **Filler** | **Angle of repose (°)** | **Bulk density (g/mL)** | **Tap density (g/mL)** | **Carr index (%)** |
|---|---|---|---|---|
| Combination of microcrystalline cellulose 102 and anhydrous calcium hydrogen phosphate | 37.44 | 0.505 | 0.700 | 28 |
| Anhydrous calcium hydrogen phosphate | 37.59 | 0.500 | 0.721 | 31 |

**Table 9. Results of compatibility between active pharmaceutical ingredient and fillers**

| **Sample placement condition** | **Sample** | **Related substance (%)** | | |
|---|---|---|---|---|
| | | **0 d** | **10 d** | **30 d** |
| **25 °C/92.5% RH** (open) | Active pharmaceutical ingredient | 0.66 | - | 0.98 |
| | Active pharmaceutical ingredient and lactose monohydrate FL100 | 0.74 | - | 0.84 |
| | Active pharmaceutical ingredient and mannitol 100SD | 0.72 | - | 0.58 |
| | Active pharmaceutical ingredient and microcrystalline cellulose 102 | 0.72 | - | 0.73 |
| | Active pharmaceutical ingredient and anhydrous calcium hydrogen phosphate | 0.73 | - | 0.91 |
| **60 °C (open)** | Active pharmaceutical ingredient | 0.66 | - | 0.66 |
| | Active pharmaceutical ingredient and lactose monohydrate FL100 | 0.74 | - | 0.64 |
| | Active pharmaceutical ingredient and mannitol 100SD | 0.72 | - | 0.62 |
| | Active pharmaceutical ingredient and microcrystalline cellulose 102 | 0.72 | - | 0.71 |
| | Active pharmaceutical ingredient and anhydrous calcium hydrogen phosphate | 0.73 | - | 0.75 |
| **40 °C/75% RH (open)** | Active pharmaceutical ingredient | 0.66 | - | 0.58 |
| | Active pharmaceutical ingredient and lactose monohydrate FL100 | 0.74 | - | 0.78 |
| | Active pharmaceutical ingredient and mannitol 100SD | 0.72 | - | 0.56 |
| | Active pharmaceutical ingredient and microcrystalline cellulose 102 | 0.72 | - | 0.59 |
| | Active pharmaceutical ingredient and anhydrous calcium hydrogen phosphate | 0.73 | - | 0.67 |
| **Light exposure (open)** | Active pharmaceutical ingredient | 0.66 | 0.64 | - |
| | Active pharmaceutical ingredient and lactose monohydrate FL100 | 0.74 | 0.64 | - |
| | Active pharmaceutical ingredient and mannitol 100SD | 0.72 | 0.67 | - |
| | Active pharmaceutical ingredient and microcrystalline cellulose 102 | 0.72 | 0.64 | - |
| | Active pharmaceutical ingredient and anhydrous calcium hydrogen phosphate | 0.73 | 0.85 | - |

### Test results:

1) It can be seen from the above experimental results that under 60 °C (open) and 40 °C/75% RH (open) conditions, all four fillers had no significant compatibility issues with the active pharmaceutical ingredient. Under light exposure (open) conditions, the impurities in the binary mixture of the active pharmaceutical ingredient and anhydrous calcium hydrogen phosphate slightly increased. However, this issue can basically be avoided through light-protected storage of the product. Therefore, anhydrous calcium hydrogen phosphate can be used for formula screening.
2) The filler screening results indicate that: in terms of powder flowability, the two formulas showed no significant difference in the powder flowability determination results and both could meet the requirements of direct powder filling; in terms of dissolution rate, the formula with anhydrous calcium hydrogen phosphate alone could reach 95% dissolution in just 30 min, while the formula with the combination of microcrystalline cellulose 102 and anhydrous calcium hydrogen phosphate could only reach a dissolution rate of 93% in 60 min, indicating that the formula using anhydrous calcium hydrogen phosphate alone has a significant advantage in dissolution performance.
3) After comprehensive evaluation of the advantages and risks of using the fillers for the product of the present invention, anhydrous calcium hydrogen phosphate alone was preferentially selected as the filler to ensure the dissolution rate; alternatively, mannitol and/or microcrystalline cellulose can also be selected as acceptable fillers.

### II. Glidant screening

Colloidal silica was used as a glidant for the following experiment.

**Table 10. Results of compatibility between active pharmaceutical ingredient and glidant**

| **Sample placement condition** | **Glidant** | **Related substance (%)** | |
|---|---|---|---|
| | | 0 d | 30 d |
| **60 °C (open)** | Active pharmaceutical ingredient | 0.66 | 0.66 |
| | Active pharmaceutical ingredient and colloidal silica 200 | 0.72 | 0.51 |
| **40 °C/75% RH (open)** | Active pharmaceutical ingredient | 0.66 | 0.58 |
| | Active pharmaceutical ingredient and colloidal silica 200 | 0.72 | 0.73 |

### Test conclusion:

It can be seen from the above experimental results that colloidal silica exhibited good compatibility with the active pharmaceutical ingredient, and thus colloidal silica was selected as the preferred glidant.

### III. Lubricant screening

Adding a proper amount of lubricant in a formulation product can reduce the friction between the material and equipment and can improve the flowability of the material. Magnesium stearate and sodium stearyl fumarate were used as lubricants for the following experiment.

The compatibility results of a binary mixture of the active pharmaceutical ingredient and each lubricant at a 10:1 weight ratio were used as the basis for lubricant type screening. The results of the compatibility between the active pharmaceutical ingredient and the lubricants are shown in the table below.

**Table 11. Results of compatibility between active pharmaceutical ingredient and lubricants**

| **Sample placement condition** | **Lubricant** | **Related substance (%)** | |
|---|---|---|---|
| | | **0 d** | **30 d** |
| **60 °C (open)** | Active pharmaceutical ingredient | 0.66 | 0.66 |
| | Active pharmaceutical ingredient and magnesium stearate | 0.62 | 0.67 |
| | Active pharmaceutical ingredient and sodium stearyl fumarate | 0.66 | 0.69 |
| **40 °C/75% RH (open)** | Active pharmaceutical ingredient | 0.66 | 0.58 |
| | Active pharmaceutical ingredient and magnesium stearate | 0.62 | 0.81 |
| | Active pharmaceutical ingredient and sodium stearyl fumarate | 0.66 | 0.80 |

### Test conclusion:

It can be seen from the above experimental results that both lubricants showed no significant changes in related substances after being placed with the active pharmaceutical ingredient under the sample placement conditions for 30 days. Therefore, it can be concluded that both lubricants had no significant compatibility issues with the active pharmaceutical ingredient; however, it was found during the preparation process that when 0.5 wt% magnesium stearate was incorporated into the formula, the issues of material sticking to the filling rod and slow dissolution were not present. Therefore, magnesium stearate was selected as the preferred lubricant.

### IV. Disintegrant screening

Theoretically, the direct powder mixing and capsule filling process does not require the addition of disintegrants to promote drug dissolution. However, in practice, considering that there may be a risk of slower dissolution due to moisture absorption and agglomeration of capsule contents during long-term product storage, a proper amount of disintegrant was incorporated into the formula to avoid this risk. Croscarmellose sodium, crospovidone and sodium carboxymethyl starch were used as disintegrants for the following experiment.

The compatibility results of a binary mixture of the active pharmaceutical ingredient and each disintegrant at a 1:1 weight ratio were used as the basis for disintegrant type screening. The results of the compatibility between the active pharmaceutical ingredient and the disintegrants are shown in the table below.

**Table 12. Results of compatibility between active pharmaceutical ingredient and disintegrants**

| **Sample placement condition** | **Disintegrant** | **Related substance (%) I** | |
|---|---|---|---|
| | | **0 d** | **30 d** |
| **60 °C (open)** | Active pharmaceutical ingredient | 0.66 | 0.66 |
| | Active pharmaceutical ingredient and croscarmellose sodium | 0.73 | 0.81 |
| | Active pharmaceutical ingredient and crospovidone XL-10 | 0.74 | 0.73 |
| | Active pharmaceutical ingredient and sodium carboxymethyl starch | 0.66 | 0.70 |
| **40 °C/75% RH (open)** | Active pharmaceutical ingredient | 0.66 | 0.58 |
| | Active pharmaceutical ingredient and croscarmellose sodium | 0.73 | 0.65 |
| | Active pharmaceutical ingredient and crospovidone XL-10 | 0.74 | 0.65 |
| | Active pharmaceutical ingredient and sodium carboxymethyl starch | 0.66 | 0.79 |

### Test conclusion:

It can be seen from the above experimental results that the selected disintegrants all had no significant compatibility issues with the active pharmaceutical ingredient. Consequently, croscarmellose sodium was selected as the disintegrant for the preferred formula.

### Example 3

### (Investigation of Particle Size of Active Pharmaceutical Ingredient)

### 1. Formula development process

The strengths of the formulation of the present invention were determined as 5 mg, 20 mg and 100 mg, with the dosage form being capsules. Auxiliary materials having good compatibility with the active pharmaceutical ingredient were selected. Preliminary process screening was first conducted using the 100 mg strength, which identified direct powder filling as the final process.

On this basis, the type and amount of filler were investigated using material flowability and product dissolution behavior as indicators, and then the formula with the 100 mg strength was preliminarily determined. Proportionally equivalent formulas were adopted for the 5 mg and 20 mg strengths. With reference to the 100 mg strength, while keeping the proportions of other auxiliary materials unchanged, only the amount of the filler, anhydrous calcium hydrogen phosphate, was adjusted to an appropriate filling amount. The formulas were preliminarily determined based on the indicators of material flowability and product dissolution behavior.

### 2. Selection of formulation process

In order to meet the requirements of clinical trial medication and pharmaceutical product prototype design, the direct powder filling process that features ease of production and scale-up and has good repeatability was considered first. However, this formulation process has relatively high requirements on the powder flowability of the mixture of the API and auxiliary materials. Therefore, on the basis of the 100 mg strength, the inventors determined the dissolution properties and micromeritic properties of the final mixed powder for formulas with different strengths of the active pharmaceutical ingredient.

### 3. Determination of particle size of active pharmaceutical ingredient

The active pharmaceutical ingredient was mechanically milled, and samples were taken at different milling time points. The particle size distribution was determined using a Malvern Mastersizer 2000 laser particle size analyzer, and the particle morphology was observed using a polarizing microscope. The particle size determination parameters are shown in Table 13, and the particle size results are shown in Table 14.

**Table 13. PSD measurement parameters**

| **Item** | **Parameter** |
|---|---|
| Analvsis mode | General |
| Sensitivity | Enhanced |
| Sample trav | General (<200 g) |
| Vibratory feeder rate | 50% |
| Dispersion air pressure | 2 Bar |
| Obscuration lower/upper limit | 10% - 20% |
| Particle type | Irregular |
| Background measurement Time | 12 s |
| Sample measurement time | 12 s |
| Measurement cycle | Each sample was measured once |

| | |
|---|---|
| Note: "PSD" is an abbreviation for particle size distribution. | |

**Table 14. Particle size distribution of active pharmaceutical ingredient**

| **Active pharmaceutical ingredient batch No.** | **Milling time** | **Particle size distribution/µm** | **PLM** |
|---|---|---|---|
| Test 0 | The active pharmaceutical ingredient was not milled | D10=35.670, D50=155.607, D90=333.563 | See FIG. 1 |
| Test 1 | Milled for 21 s | D10=2.168, D50=14.763, D90=125.775 | See FIG. 2 |
| Test 2 | Milled for 33 s | D10=1.509, D50=9.682, D90=63.594 | See FIG. 3 |
| Test 3 | Milled for 60 s | D10=1.298, D50=9.279, D90=43.137 | See FIG. 4 |

| | | | |
|---|---|---|---|
| Note: "PLM" is an abbreviation for polarizing microscope. | | | |

### 4. Effect of particle size on dissolution

Capsules of 100 mg strength were prepared using the active pharmaceutical ingredient milled to three different particle sizes as described above, and the dissolution and micromeritic properties thereof were determined. The formula composition is shown in Table 15, the dissolution results are shown in Table 16, and the micromeritic determination results are shown in Table 17.

**Table 15. Formula of 100 mg capsules**

| **Component** | **mg/capsule** | wt% | **Function** |
|---|---|---|---|
| Active pharmaceutical ingredient | 133.34* | 38.1 | Active pharmaceutical ingredient |
| Anhydrous calcium hydrogen phosphate | 200.91 | 57.4 | Filler |
| Colloidal silica 200 | 3.50 | 1.0 | Glidant |
| Croscarmellose sodium SD711 | 10.50 | 3.0 | Disintegrant |
| Magnesium stearate | 1.75 | 0.5 | Lubricant |
| Total | 350.0 | 100.0 | |
| 0# gelatin capsule | 96±6 | 1 capsule | Capsule shell |

**Table 16. Dissolution results of 100 mg capsules**

| Test No. | **API particle size (µm)** | **Dissolution (0.1 N hydrochloric acid, 900 mL, 100 rpm, N = 6, basket method)** | | | | | | |
|---|---|---|---|---|---|---|---|---|
| | | **Time (min)** | **5** | **15** | **30** | **45** | **60** | **75 (ultimate)** |
| Test 0 | D10=35.670 | Mean value (%) | 18.7 | 32.3 | 42.1 | 49.1 | 55.0 | N/A |
| | D50=155.607 | | | | | | | |
| | D90=333.563 | RSD % | 5.3 | 8.9 | 11.6 | 13.1 | 14.3 | N/A |
| Test 1 | D10=2.168 | Mean value (%) | 44 | 103 | 104 | 105 | 105 | 105 |
| | D50=14.763 | | | | | | | |
| | D90=125.775 | RSD % | 18.1 | 1.2 | 1.2 | 1.4 | 0.8 | 0.9 |
| Test 2 | D10=1.509 | Mean value (%) | 34 | 95 | 96 | 98 | 100 | 103 |
| | D50=9.682 | | | | | | | |
| | D90=63.594 | RSD % | 32.2 | 9.7 | 7.0 | 5.7 | 4.5 | 2.6 |
| Test 3 | D10=1.298 | Mean value (%) | 30 | 98 | 98 | 100 | 102 | 104 |
| | D50=8.279 | | | | | | | |
| | D90=43.137 | RSD % | 39.9 | 3.9 | 4.4 | 3.8 | 3.1 | 1.7 |

| | | | | | | | | |
|---|---|---|---|---|---|---|---|---|
| Note: "RSD" is an abbreviation for relative standard deviation. | | | | | | | | |

**Table 17. Determination results of micromeritic properties**

| **Test No.** | **API particle size (µm)** | **Angle of repose** | **Bulk density (g/mL)** | **Tap density (g/mL)** | **Carr index (%)** |
|---|---|---|---|---|---|
| Test 1 | D10=2.168 | 43.53° | 0.415 | 0.705 | 41 |
| | D50=14.763 | | | | |
| | D90=125.775 | | | | |
| Test 2 | D10=1.509 | 43.79° | 0.389 | 0.710 | 45 |
| | D50=9.682 | | | | |
| | D90=63.594 | | | | |
| Test 3 | D10=1.298 | 43.95° | 0.400 | 0.698 | 43 |
| | D50=8.279 | | | | |
| | D90=43.137 | | | | |

### 5. Test conclusion:

When the active pharmaceutical ingredient remained untreated, the dissolution of the formulation composition was incomplete. After the active pharmaceutical ingredient was mechanically milled, the particle size was significantly reduced, leading to rapid and complete dissolution. The capsules prepared with the three API batches exhibiting distinct particle size distributions all met the dissolution standards, and the flowability of all three formulas could satisfy the requirements of direct powder filling.

### Example 4

### (5 mg, 20 mg and 100 mg Capsules)

### 1. Formula composition (Table 18)

| **Strength** | **5 mg** | | **20 mg** | | **100 mg** | |
|---|---|---|---|---|---|---|
| **Component** | **mg/capsule** | wt% | **mg/capsule** | I wt% | **mg/capsule** | wt% |
| Active pharmaceutical ingredient | 6.66 | 8.33 | 26.66 | 8.33 | 133.29 | 38.08 |
| Anhydrous calcium hydrogen phosphate | 69.74 | 87.17 | 278.94 | 87.17 | 200.96 | 57.42 |
| Croscarmellose sodium | 2.40 | 3.00 | 9.60 | 3.00 | 10.50 | 3.00 |
| Colloidal silica 200 | 0.80 | 1.00 | 3.20 | 1.00 | 3.50 | 1.00 |
| Magnesium stearate | 0.40 | 0.50 | 1.60 | 0.50 | 1.75 | 0.50 |
| Total content | 80.00 | 100.00 | 320.00 | 100.00 | 350.00 | 100.00 |
| 4# gelatin capsule | 38±3 | - | - | - | - | - |
| 0# gelatin capsule | - | - | 96±6 | - | 96±6 | - |

| | | | | | | |
|---|---|---|---|---|---|---|
| Note: The strengths of the capsule formulations were calculated based on the free base of the compound of formula (I), and the active pharmaceutical ingredient was the p-toluenesulfonate monohydrate of the compound of formula (I). | | | | | | |

### 2. Production process

### 1) Weighing

According to the formula composition of the formulation of each strength described above, the active pharmaceutical ingredient, anhydrous calcium hydrogen phosphate, colloidal silica 200, croscarmellose sodium and magnesium stearate were weighed and separately placed in suitable resealable bags.

### 2) Sieving

The active pharmaceutical ingredient, anhydrous calcium hydrogen phosphate, colloidal silica 200, croscarmellose sodium and magnesium stearate were sieved through a 40-mesh sieve for later use. As a preferred technical scheme, the active pharmaceutical ingredient used in the test was first subjected to a milling pretreatment and then sieved.

### 3) Mixing

The sieved active pharmaceutical ingredient, anhydrous calcium hydrogen phosphate, colloidal silica 200 and croscarmellose sodium were placed in a hopper mixer and mixed at a rotation speed of 20 rpm for 15 min.

### 4) Lubrication

The sieved magnesium stearate was placed in the hopper mixer, and mixing was carried out at a rotation speed of 20 rpm for 5 min. After the mixing was completed, the blended powder was taken out. The properties of the blended powder are detailed in the table below.

**Table 19. Micromeritic property data of final mixed powder for 5 mg, 20 mg and 100 mg strengths**

| **Material** | **Angle of repose (°)** | **Bulk density (g/mL)** | **Tap density (g/mL)** | **Carr index (%)** |
|---|---|---|---|---|
| Active pharmaceutical ingredient | 44.66 | 0.447 | 0.740 | 40 |
| 100 mg final mixed powder | 37.59 | 0.500 | 0.721 | 31 |
| 5 mg and 25 mg final mixed powder | 33.77 | 0.446 | 0.604 | 26 |

### 5) Filling

The final mixed powder was placed in a capsule filling machine, and gelatin capsules were filled with the powder according to the respective strengths. The process parameters for each step are detailed in Table 20 below.

**Table 20. Process parameters for each step**

| **Process step** | **Process equipment** | **Process variable** | **Process parameter** | | |
|---|---|---|---|---|---|
| | | | **5 mg strength** | **20 mg strength** | **100 mg strength** |
| Pretreatment of API and auxiliary materials | 20 cm diameter stainless steel sieve | Mixture (active pharmaceutical ingredient colloidal silica 200) | 40-mesh (425 µm) | | 40-mesh (425 µm) |
| | | Anhydrous calcium hydrogen phosphate | 40-mesh (425 µm) | | 40-mesh (425 µm) |
| | | Croscarmellose sodium | 40-mesh (425 µm) | | 40-mesh (425 µm) |
| | | Magnesium stearate | 40-mesh (425 µm) | | 40-mesh (425 µm) |
| Mixing | HSD-50 hopper mixer | Mixing speed × time | 20 rpm × 15 min | | 20 rpm × 15 min |
| Lubrication | HSD-50 hopper mixer | Mixing speed × time | 20 rpm × 5 min | | 20 rpm × 5min |
| Filling | 200C-2 direct powder filling machine | Filling amount | 80.0 ×(1±7.5%) mg | 320.0 ×(1±7.5%) mg | 350.0 ×(1±7.5%) mg |
| | | Capsule length | (14.3±0.3) mm | (21.7±0.3) mm | (21.7±0.3) mm |
| | | Capsule appearance | No empty capsules, no deformation or rupture of capsules | | |

During the filling process, vacuum tube filling was used to ensure relatively stable filling amount and intact locking length and appearance; however, due to the strong adhesion of the formula material, it was found that excessive material adhered to the inner wall of the vacuum tube, so the tube needed to be cleaned to ensure normal filling operations, which made the filling process more difficult.

### 3. Formulation analysis

Since the 5 mg and 20 mg strengths adopted proportionally equivalent formulas, the capsule of 5 mg strength served as the representative for investigating relevant properties.

**Table 21. Dissolution rate results of capsules of 5 mg and 100 mg strengths**

| **Strength** | **Placement condition** | | **Dissolution rate (%)** | | | | | |
|---|---|---|---|---|---|---|---|---|
| | | | **5 min** | **15 min** | **30 min** | **45 min** | **60 min** | **75 min** |
| 5 mg | 0 d | | 75 | 96 | 100 | 101 | 100 | 103 |
| | 60 °C, open | 10 d | 86 | 98 | 98 | 100 | 100 | 100 |
| | | 30 d | 93 | 101 | 102 | 102 | 102 | 102 |
| | 40 °C/75% RH, open | 10 d | 61 | 98 | 105 | 107 | 107 | 108 |
| | | 30 d | 76 | 97 | 104 | 104 | 105 | 105 |
| | Light exposure, open | 10 d | 79 | 101 | 105 | 105 | 105 | 105 |
| 100 mg | 0 d | | 69 | 92 | 100 | 101 | 102 | 102 |
| | 60 °C, open | 10 d | 83 | 100 | 101 | 102 | 102 | 102 |
| | | 30 d | 86 | 98 | 100 | 100 | 100 | 101 |
| | 40 °C/75% RH, open | 10 d | 50 | 81 | 98 | 102 | 103 | 104 |
| | | 30 d | 52 | 78 | 93 | 100 | 103 | 104 |
| | Light exposure, open | 10 d | 62 | 92 | 102 | 105 | 106 | 107 |

**Table 22. Results of active pharmaceutical ingredient and related substance content in capsules of 5 mg strength (%)**

| **Strength** | **RRT** | **API** | **0 d** | **Condition** | | | | | | |
|---|---|---|---|---|---|---|---|---|---|---|
| | | | | **60 °C open** | | **40 °C /75% RH sealed** | | **40 °C /75% RH open** | | **Light exposure open** |
| | | | | **10d** | **30 d** | **10d** | **30 d** | **10 d** | **30 d** | **10 d** |
| | RRT=0.27 | <0.05 | <0.05 | <0.05 | <0.05 | <0.05 | <0.05 | <0.05 | <0.05 | 0.06 |
| | RRT=0.44 | 0.09 | 0.07 | 0.06 | 0.06 | 0.05 | <0.05 | 0.05 | 0.05 | <0.05 |
| | RRT=0.56 | <0.05 | <0.05 | <0.05 | <0.05 | <0.05 | <0.05 | <0.05 | <0.05 | <0.05 |
| | RRT=0.71 | 0.05 | 0.05 | 0.05 | 0.10* | 0.05 | 0.11* | 0.05 | 0.10* | 0.05 |
| | RRT=0.74 | 0.06 | 0.07 | 0.06 | | 0.07 | | 0.07 | | 0.06 |
| | RRT=0.88 | 0.06 | 0.07 | 0.07 | 0.07 | 0.07 | 0.07 | 0.07 | 0.07 | 0.07 |
| | RRT=0.97 | <0.05 | 0.05 | 0.05 | 0.06 | 0.05 | 0.06 | 0.06 | 0.06 | 0.05 |
| 5 mg | RRT=1.05 | <0.05 | 0.05 | <0.05 | 0.05 | 0.05 | 0.05 | 0.05 | 0.05 | <0.05 |
| | RRT=1.09 | <0.05 | 0.05 | <0.05 | <0.05 | <0.05 | <0.05 | 0.05 | 0.05 | <0.05 |
| | RRT=1.27 | 0.20 | 0.24 | 0.22 | 0.21 | 0.22 | 0.21 | 0.24 | 0.24 | 0.22 |
| | RRT=2.00 | 0.14 | 0.16 | 0.14 | 0.13 | 0.14 | 0.14 | 0.16 | 0.14 | 0.13 |
| | Related substance | 0.60 | 0.81 | 0.65 | 0.68 | 0.70 | 0.64 | 0.80 | 0.76 | 0.64 |
| | Content | - | 99.1 | 98.2 | 100.3 | 100.1 | 101.4 | 99.3 | 101.1 | 99.0 |
| | Chiral purity | | 100.0 | | 100.0 | | | | 100.0 | |

| | | | | | | | | | | |
|---|---|---|---|---|---|---|---|---|---|---|
| Note: *When testing the 30d samples, the chromatographic column was changed, such that two unknown impurities at RRT 0.71 and 0.74 (process impurities in API) failed to achieve baseline separation and co-eluted as a single peak. "RRT" is an abbreviation for relative retention time. | | | | | | | | | | |

**Table 23. Results of active pharmaceutical ingredient and related substance content in capsules of 100 mg strength (%)**

| **Strength** | **RRT** | **API** | **0 d** | **Condition** | | | | | | |
|---|---|---|---|---|---|---|---|---|---|---|
| | | | | **60 °C open** | | **40 °C /75% RH sealed** | | **40 °C /75% RH open** | | **Light exposure open** |
| | | | | **10 d** | **30 d** | **10 d** | **30 d** | **10d** | **30 d** | **10 d** |
| | RRT=0.27 | <0.05 | <0.05 | <0.05 | <0.05 | <0.05 | <0.05 | <0.05 | <0.05 | <0.05 |
| | RRT=0.44 | 0.09 | 0.07 | 0.06 | <0.05 | <0.05 | <0.05 | <0.05 | <0.05 | <0.05 |
| | RRT=0.56 | <0.05 | <0.05 | <0.05 | 0.06 | <0.05 | <0.05 | 0.05 | 0.05 | <0.05 |
| | RRT=0.71 | 0.05 | 0.05 | 0.05 | 0.10* | 0.05 | 0.05 | 0.05 | 0.05 | 0.05 |
| | RRT=0.74 | 0.06 | 0.07 | 0.06 | | 0.07 | 0.06 | 0.07 | 0.05 | 0.07 |
| | RRT=0.88 | 0.06 | 0.07 | 0.07 | 0.07 | 0.06 | 0.07 | 0.06 | 0.07 | 0.07 |
| 100 mg | RRT=0.97 | <0.05 | 0.05 | 0.05 | 0.05 | 0.05 | 0.06 | 0.05 | 0.06 | 0.05 |
| | RRT=1.05 | <0.05 | <0.05 | <0.05 | <0.05 | <0.05 | <0.05 | 0.05 | 0.05 | <0.05 |
| | RRT=1.09 | <0.05 | 0.05 | <0.05 | <0.05 | <0.05 | <0.05 | 0.05 | 0.05 | <0.05 |
| | RRT=1.27 | 0.20 | 0.24 | 0.21 | 0.20 | 0.21 | 0.19 | 0.23 | 0.24 | 0.23 |
| | RRT=2.00 | 0.14 | 0.15 | 0.13 | 0.11 | 0.14 | 0.11 | 0.15 | 0.14 | 0.15 |
| | Related substance | 0.60 | 0.75 | 0.63 | 0.59 | 0.58 | 0.54 | 0.76 | 0.76 | 0.62 |
| | **Content** | **-** | **101.0** | **99.6** | **100.2** | **101.2** | **100.1** | **101.1** | **101.2** | **101.3** |

| | | | | | | | | | | |
|---|---|---|---|---|---|---|---|---|---|---|
| Note: *When testing the 30d samples, the chromatographic column was changed, such that two unknown impurities at RRT 0.71 and 0.74 (process impurities in API) failed to achieve baseline separation and co-eluted as a single peak. | | | | | | | | | | |

**Table 24. Content uniformity results of 5 mg and 20 mg capsules**

| **Strength** | **Sample** | **Content (%)** | **Mean value (%)** | **SD (%)** | **Content uniformity** |
|---|---|---|---|---|---|
| **5 mg** | **Capsule 1** | 97.3 | 98.7 | 3.0 | 8.0 |
| | **Capsule 2** | 100.6 | | | |
| | **Capsule 3** | 91.4 | | | |
| | **Capsule 4** | 102.7 | | | |
| | **Capsule 5** | 99.7 | | | |
| | **Capsule 6** | 97.7 | | | |
| | **Capsule 7** | 100.7 | | | |
| | **Capsule 8** | 98.9 | | | |
| | **Capsule 9** | 98.4 | | | |
| | **Capsule 10** | 99.4 | | | |
| **20 mg** | **Capsule 1** | 97.6 | 97.7 | 1.3 | 5.2 |
| | **Capsule 2** | 97.9 | | | |
| | **Capsule 3** | 99.9 | | | |
| | **Capsule 4** | 99.5 | | | |
| | **Capsule 5** | 98.2 | | | |
| | **Capsule 6** | 97.4 | | | |
| | **Capsule 7** | 96.1 | | | |
| | **Capsule 8** | 97.0 | | | |
| | **Capsule 9** | 95.7 | | | |
| | **Capsule 10** | 97.4 | | | |

### 4. Test conclusion

(1) For the formulas of 5 mg and 100 mg strengths placed under the 60 °C/open, 40 °C/75% RH/sealed, 40 °C/75% RH/open, and light exposure/open conditions for 10 days and 30 days, no significant changes in impurities and content were observed, indicating that the chemical properties of the formulation product were stable under the accelerated conditions.
(2) For the formulas of 5 mg and 100 mg strengths placed under the 60 °C/open, 40 °C/75% RH/open, and light exposure/open conditions for 10 days and 30 days, no significant changes in dissolution were observed (since the dissolution of the samples placed under the 40 °C/75% RH/open conditions was stable, the dissolution of the samples placed under the 40 °C/75% RH/sealed conditions was not tested), indicating that the dissolution properties of the formulation product were stable under the accelerated test conditions.
(3) For the formula of 5 mg strength placed under the 60 °C/open and 40 °C/75% RH/open conditions for 30 days, no changes in chiral purity were observed, indicating that the chiral purity of the formulation product was stable under the accelerated conditions.
(4) Through the accelerated stability tests, it can be expected that the formulation product has good stability, a long shelf life and broad storage condition tolerance.
(5) The capsule weight, weight variation and content uniformity all met the requirements, indicating that the selected process is reasonable and can achieve industrialization.

**Conclusion: The capsules of 5 mg, 20 mg and 100 mg strengths were successfully filled, and all met the requirements in terms of dissolution properties, content uniformity, content and related substance. However, due to the problem of strong adhesion of the formula material, which makes the filling process more difficult, further research is still needed to obtain an industrial production method.**

### Example 5

### (Formula and Process Optimization)

In this example, formula and process screening and optimization were further conducted to obtain a pharmaceutical composition with good content uniformity and dissolution rate, which can avoid the problem of the formula material adhering to the tube or compression roller, thereby meeting the requirements of industrial production.

### 1. Formula composition (Table 25)

| **Test No.** | **5-1** | **5-2** | **5-3** | **5-4** |
|---|---|---|---|---|
| **Component** | **wt%** | **wt%** | **wt%** | **wt%** |
| **Intragranular addition material** | | | | |
| **Active pharmaceutical ingredient** | 38.1 | 38.1 | 38.1 | 38.1 |
| **Microcrystalline cellulose PH-101** | N/A | N/A | N/A | 25.45 |
| **Mannitol 200SD** | N/A | 25 | 25 | 25.45 |
| **Anhydrous calcium hydrogen phosphate (SG)** | 57.4 | 27.9 | N/A | N/A |
| **Silicified microcrystalline cellulose HD90** | N/A | N/A | 27.4 | N/A |
| **Colloidal silica 200** | 1 | 2 | 2 | N/A |
| **Hydroxypropyl cellulose EXF** | N/A | N/A | N/A | 2 |
| **Croscarmellose sodium SD-711** | 3 | 3 | 3 | 3 |
| **Magnesium stearate** | N/A | 0.5 | 0.75 | N/A |

| **Extragranular addition material** | | | | |
|---|---|---|---|---|
| **Colloidal silica 200** | N/A | N/A | N/A | 1.5 |
| **Croscarmellose sodium SD-711** | N/A | 3 | 3 | 3 |
| **Magnesium stearate** | 0.5 | 0.5 | 0.75 | 1.5 |
| **Total** | 100 | 100 | 100 | 100 |

### 2. Production process (Table 26)

| **Test No.** | **Preparation method** | **Specific procedure** |
|---|---|---|
| 5-1 | **or** Direct mixing | Prepared with reference to the production process in Example 4. |
| 5-2, 5-3 | Dry granulation | 1) The active pharmaceutical ingredient and silica were mixed and then sieved through a 40-mesh sieve; |
| | | 2) mannitol, anhydrous calcium hydrogen phosphate (or silicified microcrystalline cellulose) and croscarmellose sodium were mixed and then sieved through a 40-mesh sieve; |
| | | 3) magnesium stearate (intragranular addition) was added for mixing; |
| | | 4) the intragranular mixed material described above was subjected to dry granulation; |
| | | 5) the croscarmellose sodium that had passed through the 40-mesh sieve was mixed with the dry granules prepared in the previous step; |
| | | 6) the magnesium stearate that had passed through the 40-mesh sieve was further added for mixing; and |
| | | 7) filling was then performed. |
| 5-4 | Wet granulation | 1) The active pharmaceutical ingredient and microcrystalline cellulose were mixed; |
| | | 2) the mixture of the active pharmaceutical ingredient and microcrystalline cellulose, mannitol, and croscarmellose sodium (intragranular addition) were separately sieved through a 40-mesh sieve; |
| | | 3) a binder was prepared at a ratio of hydroxypropyl cellulose/(water + hydroxypropyl cellulose) = 8% (w/w); |
| | | 4) wet granulation was performed; |
| | | 5) drying was performed; |
| | | 6) colloidal silica and croscarmellose sodium (extragranular addition) were mixed and then sieved through a 40-mesh sieve; |
| | | 7) the magnesium stearate that had passed through the 40-mesh sieve was directly added into the mixing bucket containing the extragranular addition materials for lubrication, or the magnesium stearate that had passed through the 40-mesh sieve was first mixed with a small portion of extragranular addition mixed granules and then added into the mixing bucket containing the extragranular addition materials for lubrication; and |
| | | 8) filling was then performed. |

The specific results of the micromeritic properties of the final mixed powders and the content uniformity of capsules are shown in the following tables.

**Table 27. Micromeritic property data of final mixed powders**

| **Test No.** | **Bulk density (g/mL)** | **Tap density (g/mL)** | **Carr index (%)** |
|---|---|---|---|
| **5-1** | 0.470 | 0.720 | 34.7 |
| **5-2** | 0.527 | 0.782 | 32.6 |
| **5-3** | 0.555 | 0.827 | 32.9 |
| **5-4** | 0.396 | 0.545 | 27.3 |

**Table 28. Results of content uniformity of capsules**

| **Test No.** | **Sample** | **Content (%)** | **Mean value (%)** | **SD (%)** |
|---|---|---|---|---|
| **5-1** | **Capsule 1** | 97.9 | 98.1 | 0.9 |
| | **Capsule 2** | 97.2 | | |
| | **Capsule 3** | 98.3 | | |
| | **Capsule 4** | 96.1 | | |
| | **Capsule 5** | 98.0 | | |
| | **Capsule 6** | 98.7 | | |
| | **Capsule 7** | 97.8 | | |
| | **Capsule 8** | 98.7 | | |
| | **Capsule 9** | 98.9 | | |
| | **Capsule 10** | 98.1 | | |
| | **Capsule 11** | 99.3 | | |
| **5-4** | **Capsule 1** | 99.9 | 100.0 | 0.2 |
| | **Capsule 2** | 100.4 | | |
| | **Capsule 3** | 100.1 | | |
| | **Capsule 4** | 99.7 | | |
| | **Capsule 5** | 100.1 | | |
| | **Capsule 6** | N/A | | |
| | **Capsule 7** | N/A | | |
| | **Capsule 8** | N/A | | |
| | **Capsule 9** | N/A | | |
| | **Capsule 10** | N/A | | |
| | **Capsule 11** | N/A | | |
| **Note** | "N/A" **means not tested.** | | | |

### 3. Test conclusion

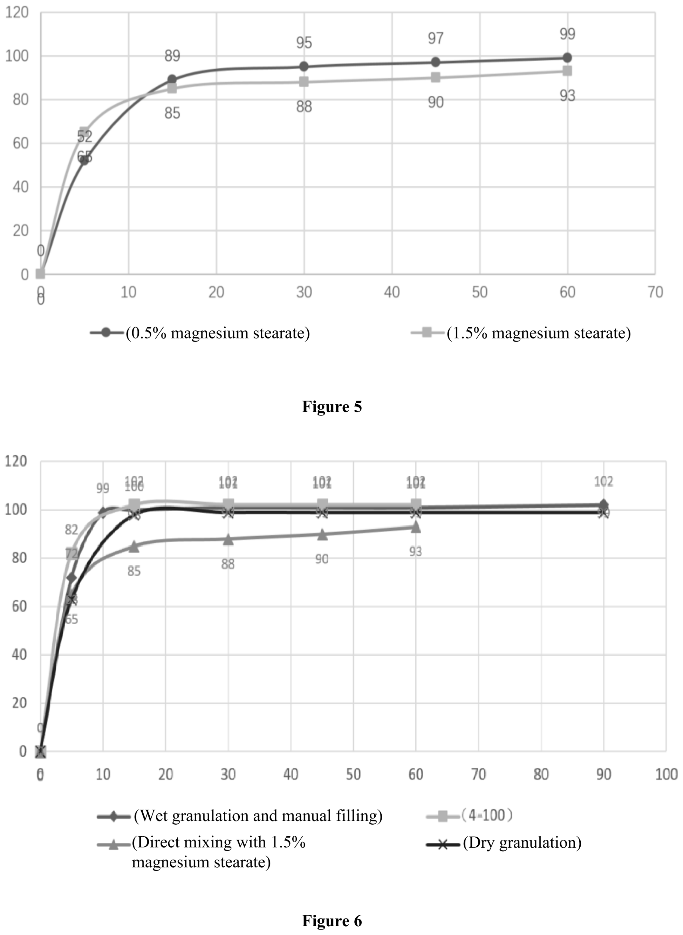

(1) Pharmaceutical composition 5-1 was prepared by direct mixing. During the process, it was observed that after 20 min of running, there was no adhesion to the filling needle, but the material had poor flowability, so that the filling amount fluctuated relatively greatly and exceeded the allowable limit; subsequently, 1.0% magnesium stearate (bringing the total proportion to about 1.5%) was additionally added to the remaining material for a second filling attempt; after another 20 min of running, there was no adhesion to the filling needle, but the filling amount still exceeded the allowable limit. Capsules closest to the target value were selected from both filling runs and sent for dissolution testing. The results, as shown in FIG. 5, demonstrated that the formula with 1.5% magnesium stearate did not achieve complete drug release during dissolution, indicating that in direct mixing preparations, formulas containing about 1.5% magnesium stearate may impact the dissolution.
(2) Pharmaceutical composition 5-2 was prepared by dry granulation. During the process, it was observed that a small amount of material adhered to the compression roller with strong adhesion force during the dry granulation stage, requiring regular cleaning. After capsule filling, samples were sent for dissolution testing. The results, as shown in FIG. 6, demonstrated that the dissolution performance was similar to that of the formula of 100 mg strength in Example 4 (4-100).
(3) For pharmaceutical composition 5-3, the filler was changed from anhydrous calcium hydrogen phosphate to silicified microcrystalline cellulose, and the proportion of magnesium stearate was increased from 0.5% to 0.75% (for both intragranular addition and extragranular addition). During the preparation process, it was observed that a small amount of material still adhered to the compression roller with strong adhesion force during the dry granulation stage.
(4) Pharmaceutical composition 5-4 was prepared by wet granulation. During the process, it was observed that the wet granulation process was relatively smooth and the material had relatively good flowability. After capsule filling, samples were sent for dissolution testing. The results, as shown in FIG. 6, demonstrated that the dissolution performance was similar to that of the formula of 100 mg strength in Example 4 (4-100).

In conclusion, compared with direct mixing or dry granulation, the formula material obtained by wet granulation showed a significant reduction in Carr index and substantial improvement in material flowability, and exhibited good content uniformity.

### Example 6

### (50 mg, 60 mg and 100 mg Capsules)

### 1. Formula composition (Table 29)

| **Test No.** | **Unit proportion** | **6-1 (50 mg)** | **6-2 (60 mg)** | **6-3 (100 mg)** |
|---|---|---|---|---|
| **Component** | **wt%** | **wt%** | **wt%** | **wt%** |
| **Intragranular addition material** | | | | |
| **Active pharmaceutical ingredient** | 38.10 | 66.68 | 80.01 | 133.35 |
| **Microcrystalline cellulose PH-101** | 25.45 | 44.54 | 53.44 | 89.08 |
| **Mannitol 200SD** | 25.45 | 44.54 | 53.44 | 89.08 |
| **Hydroxypropyl cellulose EXF** | 2.00 | 3.50 | 4.20 | 7.00 |
| **Croscarmellose sodium SD-711** | 3.00 | 5.25 | 6.30 | 10.50 |

| **Extragranular addition material** | | | | |
|---|---|---|---|---|
| **Colloidal silica 200** | 1.50 | 2.62 | 3.15 | 5.25 |
| **Croscarmellose sodium SD-711** | 3.00 | 5.25 | 6.30 | 10.50 |
| **Magnesium stearate** | 1.50 | 2.62 | 3.15 | 5.25 |
| **Total** | 100 | 175.0 | 210.0 | 350.0 |

### 2. Production process

The preparation was conducted with reference to the production process of Test No. 5-4 in Example 5. The specific results of capsule content, moisture, impurities, content uniformity and dissolution are shown in the following tables.

**Table 30. Summary of capsule content, moisture and impurity data**

| **Test No.** | **Content (%)** | **Moisture (%)** | **Capsule product impurities** | **API impurities** |
|---|---|---|---|---|
| **6-1** | 102.3 | 2.5 | RRT 2.00: 0.05% | Single impurity < 0.05% |
| | | | Total impurities: 0.05% | Total impurities < 0.05% |
| **6-2** | 100.5 | 2.2 | RRT 2.00: 0.05% | Single impurity < 0.05% |
| | | | Total impurities: 0.05% | Total impurities < 0.05% |
| **6-3** | 101.4 | 2.4 | RRT 2.00: 0.05% | Single impurity < 0.05% |
| | | | Total impurities: 0.05% | Total impurities < 0.05% |

**Table 31. Results of content uniformity of capsules**

| **Test No.** | **Sample** | **Content (%)** | **Mean value (%)** | **SD (%)** | **A+2.2S** |
|---|---|---|---|---|---|
| **6-1** | **Capsule 1** | 97.8 | 101.6 | 2.8 | 7.9 |
| | **Capsule 2** | 100.9 | | | |
| | **Capsule 3** | 103.8 | | | |
| | **Capsule 4** | 106.5 | | | |
| | **Capsule 5** | 102.2 | | | |
| | **Capsule 6** | 96.4 | | | |
| | **Capsule 7** | 102.7 | | | |
| | **Capsule 8** | 101.0 | | | |
| | **Capsule 9** | 102.1 | | | |
| | **Capsule 10** | 102.3 | | | |
| **6-2** | **Capsule 1** | 100.0 | 100.0 | 1.2 | 2.7 |
| | **Capsule 2** | 99.8 | | | |
| | **Capsule 3** | 99.3 | | | |
| | **Capsule 4** | 99.5 | | | |
| | **Capsule 5** | 98.9 | | | |
| | **Capsule 6** | 98.3 | | | |
| | **Capsule 7** | 101.1 | | | |
| | **Capsule 8** | 99.2 | | | |
| | **Capsule 9** | 102.0 | | | |
| | **Capsule 10** | 101.6 | | | |
| **6-3** | **Capsule 1** | 96.5 | 100.4 | 2.2 | 5.3 |
| | **Capsule 2** | 99.1 | | | |
| | **Capsule 3** | 99.2 | | | |
| | **Capsule 4** | 101.9 | | | |
| | **Capsule 5** | 103.3 | | | |
| | **Capsule 6** | 101.4 | | | |
| | **Capsule 7** | 100.3 | | | |
| | **Capsule 8** | 101.6 | | | |
| | **Capsule 9** | 102.8 | | | |
| | **Capsule 10** | 97.5 | | | |
| **Note** | "N/A" **means not tested.** | | | | |

**Table 32. Dissolution results**

| **Test No.** | **No./Time point** | **5 min** | **15 min** | **30 min** | **45 min** | **60 min** | **Capsule weight** |
|---|---|---|---|---|---|---|---|
| **6-1** | **1** | 96 | 103 | 103 | 103 | 103 | 237.07 |
| | **2** | 93 | 102 | 102 | 102 | 102 | 238.77 |
| | **3** | 86 | 101 | 101 | 101 | 101 | 237.67 |
| | **4** | 91 | 103 | 102 | 102 | 102 | 237.96 |
| | **5** | 93 | 100 | 100 | 100 | 100 | 236.09 |
| | **6** | 93 | 104 | 104 | 104 | 104 | 236.55 |
| | **Mean value %** | 92 | 102 | 102 | 102 | 102 | N/A |
| | **Relative standard deviation %** | 3.6 | 1.3 | 1.2 | 1.2 | 1.2 | |
| **6-2** | **1** | 90 | 98 | 98 | 98 | 98 | 280.87 |
| | **2** | 87 | 100 | 100 | 100 | 100 | 286.81 |
| | **3** | 81 | 99 | 99 | 99 | 99 | 284.36 |
| | **4** | 86 | 100 | 101 | 101 | 101 | 288.82 |
| | **5** | 88 | 99 | 99 | 99 | 99 | 284.59 |
| | **6** | 90 | 99 | 99 | 99 | 99 | 284.99 |
| | **Mean value %** | 87 | 99 | 99 | 99 | 99 | N/A |
| | **Relative standard deviation %** | 3.9 | 0.8 | 0.9 | 1.0 | 0.9 | |
| **6-3** | **1** | 87 | 98 | 98 | 98 | 98 | 437.84 |
| | **2** | 78 | 100 | 100 | 100 | 101 | 450.42 |
| | **3** | 90 | 101 | 101 | 101 | 101 | 448.16 |
| | **4** | 88 | 101 | 101 | 101 | 101 | 450.03 |
| | **5** | 92 | 102 | 102 | 102 | 102 | 448.58 |
| | **6** | 88 | 99 | 99 | 99 | 99 | 446.97 |
| | **Mean value %** | 87 | 100 | 100 | 100 | 100 | N/A |
| | **Relative standard deviation %** | 5.8 | 1.7 | 1.6 | 1.6 | 1.6 | |
| **Note** | "N/A" **means not calculated.** | | | | | | |

### 3. Test conclusion

It can be seen from the above test results that for the formulas of different strengths provided in this example, the moisture results were reported values; the impurity results showed that no new abnormal impurities were found in any batch compared with the API, and the requirements of single impurity ≤ 0.2% and total impurity ≤ 2.0% were met for all the batches. The content uniformity results all met the requirements of A + 2.2S ≤ 15.0 and AV ≤ 15.0, which were in line with the acceptance standards of both *Chinese Pharmacopoeia* and *United States Pharmacopoeia,* demonstrating good dissolution.

All documents mentioned in the present invention are incorporated herein by reference, just as each document is individually cited as a reference. In addition, it should be understood that various modifications or changes may be made by those skilled in the art after reading the above content of the present invention, and these equivalent forms also fall within the scope defined by the appended claims of the present application.

## Claims

1. A pharmaceutical composition, comprising a free base of a compound of formula (I) or an acidic salt thereof as an active ingredient, a pharmaceutically acceptable filler and an additional pharmaceutically acceptable carrier, 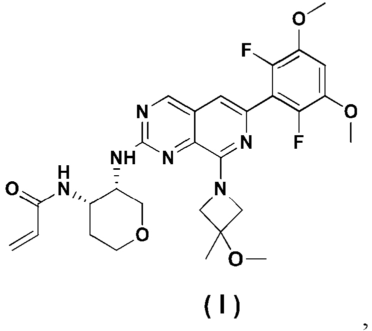 wherein the pharmaceutically acceptable filler is one or more of calcium hydrogen phosphate, mannitol, lactose, microcrystalline cellulose and silicified microcrystalline cellulose.

2. The pharmaceutical composition according to claim 1, wherein the content of the active ingredient is 0.1-60 wt% of the total weight of the pharmaceutical composition, as calculated based on a *p*-toluenesulfonate monohydrate of the compound of formula (I);
**preferably,** the content of the active ingredient is 1.0-50 wt% of the total weight of the pharmaceutical composition, as calculated based on the *p*-toluenesulfonate monohydrate of the compound of formula (I); and
**more preferably,** the content of the active ingredient is 5.0-40 wt% of the total weight of the pharmaceutical composition, as calculated based on the *p*-toluenesulfonate monohydrate of the compound of formula (I).

3. The pharmaceutical composition according to claim 1, wherein the additional pharmaceutically acceptable carrier is one or more of a glidant, a lubricant, a binder and a disintegrant.

4. The pharmaceutical composition according to claim 3, wherein the glidant is colloidal silica, and the content of the glidant is 0.1-5.0 wt% of the total weight of the pharmaceutical composition;
**preferably,** the content of the glidant is 0.5-4.0 wt% of the total weight of the pharmaceutical composition; and
**more preferably,** the content of the glidant is 0.8-2.0 wt% of the total weight of the pharmaceutical composition.

5. The pharmaceutical composition according to claim 3, wherein the lubricant is magnesium stearate and/or sodium stearyl fumarate, and the content of the lubricant is 0.1-5.0 wt% of the total weight of the pharmaceutical composition;
**preferably,** the content of the lubricant is 0.2-3.0 wt% of the total weight of the pharmaceutical composition;
**more preferably,** the content of the lubricant is 0.3-1.5 wt% of the total weight of the pharmaceutical composition; and
**more preferably,** the content of the lubricant is 0.3-1.0 wt% of the total weight of the pharmaceutical composition.

6. The pharmaceutical composition according to claim 3, wherein the binder is hydroxypropyl cellulose, methyl cellulose, hydroxypropyl methyl cellulose, polyvinylpyrrolidone, povidone and/or sodium carboxymethyl starch, and the content of the binder is 0.0-20.0 wt% of the total weight of the pharmaceutical composition;
**preferably,** the content of the binder is 0.0-10.0 wt% of the total weight of the pharmaceutical composition; and
**more preferably,** the content of the binder is 0.0-4.0 wt% of the total weight of the pharmaceutical composition.

7. The pharmaceutical composition according to claim 3, wherein the disintegrant is croscarmellose sodium and/or sodium carboxymethyl starch, and the content of the disintegrant is 0.5-20.0 wt% of the total weight of the pharmaceutical composition;
**preferably,** the content of the disintegrant is 1.0-10.0 wt% of the total weight of the pharmaceutical composition; and
**more preferably,** the content of the disintegrant is 2.0-6.0 wt% of the total weight of the pharmaceutical composition.

8. The pharmaceutical composition according to claim 1, wherein the pharmaceutically acceptable filler is anhydrous calcium hydrogen phosphate.

9. The pharmaceutical composition according to claim 1, wherein the pharmaceutically acceptable filler is one or more of mannitol and microcrystalline cellulose; and
**preferably,** the pharmaceutically acceptable filler is a mixture of mannitol and microcrystalline cellulose.

10. The pharmaceutical composition according to claim 3, wherein the content of the pharmaceutically acceptable filler is 1.0-98.0 wt% of the total weight of the pharmaceutical composition;
**preferably,** the content of the pharmaceutically acceptable filler is 20.0-95.0 wt% of the total weight of the pharmaceutical composition; and
**more preferably,** the content of the pharmaceutically acceptable filler is 40.0-90.0 wt% of the total weight of the pharmaceutical composition.

11. The pharmaceutical composition according to any one of claims 1-10, wherein a unit dosage form of the pharmaceutical composition comprises the active ingredient, a glidant, a lubricant, a binder and a disintegrant,
wherein the content of the active ingredient is 5.0-40.0 wt% of the total weight of the pharmaceutical composition, and/or
the content of the glidant is 0.8-2.0 wt% of the total weight of the pharmaceutical composition, and/or
the content of the lubricant is 0.3-1.0 wt% of the total weight of the pharmaceutical composition, and/or
the content of the binder is 0.0-4.0 wt% of the total weight of the pharmaceutical composition, and/or
the content of the disintegrant is 2.0-6.0 wt% of the total weight of the pharmaceutical composition.

12. The pharmaceutical composition according to claim 1, wherein a unit dosage form of the pharmaceutical composition comprises the active ingredient, colloidal silica, magnesium stearate, hydroxypropyl cellulose and croscarmellose sodium,
wherein the content of the active ingredient is 5.0-40.0 wt% of the total weight of the pharmaceutical composition, and/or
the content of colloidal silica is 0.8-2.0 wt% of the total weight of the pharmaceutical composition, and/or
the content of magnesium stearate is 0.3-1.0 wt% of the total weight of the pharmaceutical composition, and/or
the content of hydroxypropyl cellulose is 0.0-4.0 wt% of the total weight of the pharmaceutical composition, and/or
the content of croscarmellose sodium is 2.0-6.0 wt% of the total weight of the pharmaceutical composition.

13. The pharmaceutical composition according to claim 1, wherein when the sum of the contents of the components in the pharmaceutical composition is less than 100%, the content of the filler is adjusted such that the sum of the contents of the components in the pharmaceutical composition is 100 wt%.

14. The pharmaceutical composition according to claim 1, wherein the active ingredient is an anhydrate, a hydrate or a solvate of the free base of the compound of formula (I) or the acidic salt thereof;
**preferably,** the active ingredient is a solvate of the free base of the compound of formula (I) or the acidic salt thereof, wherein the solvent is selected from the group consisting of organic solvents including alcohols, chloroalkanes, ketones, ethers, cyclic ethers, esters, alkanes, cycloalkanes, benzenes, amides and sulfoxides, and a mixture thereof and an aqueous solution thereof;
**more preferably,** the active ingredient is a solvate of the free base of the compound of formula (I) or the acidic salt thereof, wherein the solvent is selected from the group consisting of methanol, ethanol, *n*-propanol, isopropanol, dichloromethane, acetonitrile, acetone, 1,4-dioxane, tetrahydrofuran, *N,N*-dimethylformamide, ethyl acetate, isopropyl acetate, methyl *tert-*butyl ether and 2-methoxyethyl ether, and a mixture thereof and an aqueous solution thereof;
**preferably,** the active ingredient is an anhydrate or a hydrate of the free base of the compound of formula (I) or the acidic salt thereof;
**more preferably,** the active ingredient is an anhydrate of the free base of the compound of formula (I) or the acidic salt thereof;
**more preferably,** the active ingredient is a hydrate of the free base of the compound of formula (I) or the acidic salt thereof, wherein the hydrate comprises 1-3 water molecules per molecule;
**more preferably,** the active ingredient is a hydrate of *p*-toluenesulfonate of the compound of formula (I), wherein the hydrate comprises 1-3 water molecules per molecule; and
**more preferably,** the active ingredient is a hydrate of *p*-toluenesulfonate of the compound of formula (I), wherein the hydrate comprises 1 water molecule per molecule.

15. The pharmaceutical composition according to claim 14, wherein the active ingredient is a *p*-toluenesulfonate monohydrate of the compound of formula (I).

16. The pharmaceutical composition according to claim 15, wherein an X-ray powder diffraction pattern of the *p*-toluenesulfonate monohydrate of the compound of formula (I) comprises 5 or more peaks at diffraction angles (2θ) selected from the group consisting of 9.02±0.2°, 10.60±0.2°, 12.04±0.2°, 12.72±0.2°, 15.32±0.2°, 17.18±0.2°, 17.68±0.2°, 18.56±0.2°, 19.60±0.2°, 20.60±0.2°, 21.22±0.2°, 22.90±0.2°, 23.82±0.2°, 24.12±0.2°, 24.60±0.2°, 24.90±0.2°, 25.58±0.2°, 27.78±0.2°, 29.04±0.2°, 31.48±0.2°, 36.60±0.2° and 38.16±0.2°.

17. The pharmaceutical composition according to claim 15, wherein the active ingredient is a *p*-toluenesulfonate monohydrate of the compound of formula (I), having a particle size (D90) of 5-330 µm, **preferably** having a particle size (D90) of 10-200 µm, and **more preferably** having a particle size (D90) of 20-150 µm.

18. The pharmaceutical composition according to claim 15, wherein the active ingredient is a *p*-toluenesulfonate monohydrate of the compound of formula (I), having a particle size (D50) of 2-200 µm, **preferably** having a particle size (D50) of 4-100 µm, **more preferably** having a particle size (D50) of 6-50 µm, and **more preferably** having a particle size (D50) of 6-25 µm.

19. The pharmaceutical composition according to claim 1, wherein the pharmaceutical composition is obtained by a direct powder filling, dry granulation or wet granulation process; and **preferably,** the pharmaceutical composition is obtained by a wet granulation process.

20. The pharmaceutical composition according to claim 1, wherein the pharmaceutical composition is in a capsule or a tablet form.

21. The pharmaceutical composition according to claim 1, wherein a unit dosage form of the pharmaceutical composition comprises 1-500 mg of the active ingredient, as calculated based on the free base of the compound of formula (I);
**preferably,** the unit dosage form of the pharmaceutical composition comprises 1-200 mg of the active ingredient, as calculated based on the free base of the compound of formula (I); and
**more preferably,** the unit dosage form of the pharmaceutical composition comprises 1 mg, 5 mg, 20 mg, 50 mg, 60 mg, 80 mg, 100 mg or 200 mg of the active ingredient, as calculated based on the free base of the compound of formula (I).

22. A preparation method for the pharmaceutical composition according to claim 1, comprising the following step(s):
mixing the free base of the compound of formula (I) or the acidic salt thereof as the active ingredient with the pharmaceutically acceptable filler to obtain the pharmaceutical composition, 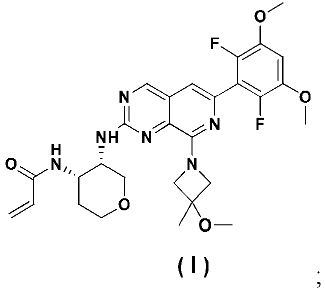 and
**optionally,** adding the additional pharmaceutically acceptable carrier before, during or after the mixing; and
**optionally,** subjecting the pharmaceutical composition prepared above to powder filling, dry granulation or wet granulation to obtain a capsule or tableting to obtain a tablet.

23. The preparation method according to claim 22, wherein the additional pharmaceutically acceptable carrier is one or more of a glidant, a lubricant, a binder and a disintegrant.

24. The preparation method according to claim 22, comprising the following step(s):
mixing a *p*-toluenesulfonate monohydrate of the compound of formula (I) with calcium hydrogen phosphate, croscarmellose sodium, colloidal silica and/or an additional pharmaceutically acceptable carrier to obtain a pharmaceutical composition; and
**optionally,** further mixing magnesium stearate and/or sodium stearyl fumarate with the aforementioned pharmaceutical composition to obtain the pharmaceutical composition.

25. The preparation method according to claim 22, comprising the following step(s):
mixing a *p*-toluenesulfonate monohydrate of the compound of formula (I) with mannitol, microcrystalline cellulose, a first portion of croscarmellose sodium and/or an additional pharmaceutically acceptable carrier to obtain a pharmaceutical composition; and
**optionally,** adding an aqueous solution of hydroxypropyl cellulose, performing wet granulation, and then drying;
**optionally,** adding colloidal silica and a second portion of croscarmellose sodium to further mix with dried granules obtained in the previous step; and
**optionally,** adding magnesium stearate and performing final mixing to obtain the pharmaceutical composition.

26. The preparation method according to claim 22, wherein before and/or after the mixing, the components of the pharmaceutical composition are sieved individually or as a mixture through a 30- to 100-mesh sieve, **preferably** a 30- to 50-mesh sieve, and **more preferably** a 40-mesh sieve; particularly, the active ingredient of the pharmaceutical composition is separately subjected to a milling pretreatment before the mixing.

27. The preparation method according to claim 22, wherein the mixing of the components of the pharmaceutical composition is performed at a speed of 10-100 rpm, **preferably** 10-50 rpm, and **more preferably** 20-30 rpm.

28. Use of the pharmaceutical composition according to claim 1 in the preparation of a medicament as an FGFR4 inhibitor.

29. Use of the pharmaceutical composition according to claim 1 in the preparation of a medicament for treating FGFR4-associated liver cancer, prostate cancer, pancreatic cancer, esophageal cancer, gastric cancer, lung cancer, breast cancer, ovarian cancer, colon cancer, skin cancer, glioblastoma or rhabdomyosarcoma.

30. A method for treating FGFR4-associated liver cancer, prostate cancer, pancreatic cancer, esophageal cancer, gastric cancer, lung cancer, breast cancer, ovarian cancer, colon cancer, skin cancer, glioblastoma or rhabdomyosarcoma, comprising administering to a patient in need thereof a therapeutically effective amount of the pharmaceutical composition according to claim 1.
